# EUROPEAN PATENT SPECIFICATION

(11) **EP 3 653 129 B1**
(45) Date of publication and mention of the grant of the patent: **07.12.2022**
(21) Application number: 19203360.3
(22) Date of filing: 15.10.2019
(51) Int. Cl.: A61B 8/06, A61B 8/00, A61B 8/08

(54) **ULTRASOUND IMAGING APPARATUS AND OPERATING METHOD THEREOF**
ULTRASCHALLBILDGEBUNGSVORRICHTUNG UND BETRIEBSVERFAHREN DAFÜR
APPAREIL D'IMAGERIE À ULTRASONS ET PROCÉDÉ DE FONCTIONNEMENT ASSOCIÉ

(30) Priority: 15.11.2018 KR 20180141134
(43) Date of publication of application: 20.05.2020
(73) Proprietor: Samsung Medison Co., Ltd., Hongcheon-gun, Gangwon-do 25108 (KR)
(72) Inventor: Kwon, Sora, Gyeonggi-do (KR); Ma, Hyemin, Gyeonggi-do (KR); Rhee, Daekyun, Gyeonggi-do (KR); Yang, Eunho, Gyeonggi-do (KR)
(74) Representative: Hylarides, Paul Jacques

(56) References cited:
- WO-A1-2018/051578
- US-A1- 2004 167 416
- US-A1- 2007 055 153
- US-A1- 2008 114 240
- US-A1- 2012 253 195
- US-A1- 2018 129 782
- MYERS J G ET AL: "Color Doppler velocity accuracy proximal to regurgitant orifices: influence of orifice aspect ratio", ULTRASOUND IN MEDICINE AND BIOLOGY, NEW YORK, NY, US, vol. 25, no. 5, 1 June 1999 (1999-06-01), pages 771-792, XP027326282, ISSN: 0301-5629 [retrieved on 1999-06-01]

## Description

### BACKGROUND

### 1. Field

The disclosure relates to ultrasound imaging apparatuses, and more particularly, to ultrasound imaging apparatuses for automatically saving an ultrasound image.

### 2. Description of Related Art

Ultrasound imaging apparatuses transmit ultrasound signals generated by transducers of a probe to an object and detect information about echo signals reflected from the object, thereby obtaining at least one image of an internal part, for example, soft tissue or blood flow, of the object.

Furthermore, an ultrasound imaging apparatus may store ultrasound images acquired via a probe. The ultrasound imaging apparatus may store ultrasound images as a cine image. A cine image is a series of cross-sectional images of an internal part of an object, which are arranged sequentially over time, and may be used to examine movement of the internal part of the object. In general, a cine image of the heart may be used to determine the presence of cardiac dysfunction such as a reversal of cardiac blood flow.

Selective saving of ultrasound images is known from the documents US 2004/167416,US 2012/253195 and US 2018/129782.

### SUMMARY

The invention is defined in the independent claims. Preferred embodiments are provided in the dependent claims.

Provided are ultrasound imaging apparatuses and operating methods thereof, which are capable of automatically saving an ultrasound image. In detail, provided are methods of automatically saving an ultrasound image when an ultrasound image obtained in real-time satisfies a preset condition without a user performing any specific operation for saving the ultrasound image.

Additional aspects will be set forth in part in the description which follows and, in part, will be apparent from the description, or may be learned by practice of the presented embodiments.

In accordance with an aspect of the disclosure, an ultrasound imaging apparatus capable of automatically saving an ultrasound image of an object includes: a probe configured to acquire the ultrasound image of the object; a memory storing the ultrasound image of the object; and at least one processor configured to set a condition for automatically saving the ultrasound image of the object, set an ultrasound image acquisition and save mode for automatically acquiring and saving the ultrasound image of the object according to the condition, and automatically acquire and save, when a real-time ultrasound image of the object satisfies the condition, the ultrasound image of the object based on the ultrasound image acquisition and save mode.

According to the invention, the condition includes a condition where a velocity of blood flow in a region of interest in the real-time ultrasound image of the object is greater than a particular value.

Additionally the condition may further include a condition where an area of a valve orifice in a region of interest in the real-time ultrasound image of the object is greater than a particular value.

Additionally the condition may further include a condition where a size of a regurgitant jet area in a region of interest in the real-time ultrasound image of the object is greater than a particular value.

The at least one processor may be further configured to set the ultrasound image acquisition and save mode by setting whether to acquire the ultrasound image of the object at a predetermined frame rate or higher and save the acquired ultrasound image of the object.

The at least one processor may be further configured to play back, when the saved ultrasound image is an ultrasound acquired and saved at the predetermined frame rate or higher, the saved ultrasound image at a speed less than a speed of acquiring the ultrasound image.

The at least one processor is further configured to set the ultrasound image acquisition and save mode by obtaining an ultrasound image of the object and setting whether to acquire and save an enlarged version of an image of a portion corresponding to a region of interest in the obtained ultrasound image.

The at least one processor may be further configured to set the ultrasound image acquisition and save mode by setting a time period during which the ultrasound image of the object to be automatically saved is acquired.

The at least one processor may be further configured to acquire, when the real-time ultrasound image of the object satisfies the condition, the ultrasound image of the object during the set time period based on the ultrasound image acquisition and save mode and save the ultrasound image acquired during the set time period as a cine image.

The at least one processor may be further configured to add at least one or a combination of an annotation and a body marker to the acquired ultrasound image.

The at least one processor may be further configured to automatically execute, when the ultrasound image is acquired and saved, a function related to the saved ultrasound image, and the function related to the saved ultrasound image may include at least one or a combination of a diagnostic function with respect to the object in the saved ultrasound image and a measurement function with respect to the saved ultrasound image.

In accordance with another aspect of the disclosure, a method of operating an ultrasound imaging apparatus includes: setting a condition for automatically saving an ultrasound image of an object; setting an ultrasound image acquisition and save mode for automatically acquiring and saving the ultrasound image of the object according to the condition; and automatically acquiring and saving, when a real-time ultrasound image of the object satisfies the condition, the ultrasound image of the object based on the ultrasound image acquisition and save mode.

In accordance with another aspect of the disclosure, a computer program product includes a recording medium having stored therein program instructions that, when executed by a processor, cause the processor to perform a method of automatically saving an ultrasound image of an object, the method including: setting a condition for automatically saving the ultrasound image of the object; setting an ultrasound image acquisition and save mode for automatically acquiring and saving the ultrasound image of the object according to the condition; and automatically acquiring and saving, when a real-time ultrasound image of the object satisfies the condition, the ultrasound image of the object based on the ultrasound image acquisition and save mode.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a block diagram illustrating an ultrasound imaging apparatus according to an exemplary embodiment;
FIGS. 2A, 2B, and 2C are diagrams respectively illustrating an ultrasound imaging apparatus according to an exemplary embodiment;
FIG. 3 illustrates a structure of an ultrasound imaging apparatus according to some embodiments;
FIG. 4 is a flowchart of a method, performed by an ultrasound imaging apparatus, of automatically saving an ultrasound image, according to some embodiments;
FIG. 5 illustrates an example in which an ultrasound imaging apparatus automatically saves an ultrasound image when a condition for automatically saving an ultrasound image is set based on a velocity of blood flow, according to some embodiments;
FIG. 6 illustrates an example in which an ultrasound imaging apparatus automatically saves an ultrasound image when a condition for automatically saving an ultrasound image is set based on an area of a valve orifice, according to some embodiments;
FIG. 7 illustrates an example in which an ultrasound imaging apparatus automatically saves an ultrasound image when a condition for automatically saving an ultrasound image is set based on a size of a regurgitant jet area, according to some embodiments;
FIG. 8 illustrates an example in which an ultrasound imaging apparatus automatically acquires an ultrasound image at a predetermined frame rate or higher and saves the ultrasound image, according to some embodiments;
FIG. 9 illustrates an example in which an ultrasound imaging apparatus automatically acquires and saves an ultrasound image obtained by enlarging a portion corresponding to a region of interest (ROI), according to some embodiments;
FIG. 10 is a flowchart of a method, performed by an ultrasound imaging apparatus, of automatically saving an ultrasound image by adding an annotation or body marker thereto, according to some embodiments;
FIG. 11 illustrates an example in which an ultrasound imaging apparatus adds an annotation to an automatically saved ultrasound image, according to some embodiments;
FIG. 12 illustrates an example in which an ultrasound imaging apparatus adds a body marker to an automatically saved ultrasound image, according to some embodiments;
FIG. 13 is a flowchart of a method, performed by an ultrasound imaging apparatus, of automatically saving an ultrasound image and automatically executing a function related to the saved ultrasound image, according to some embodiments;
FIG. 14 illustrates an example in which an ultrasound imaging apparatus sets a condition for automatically saving an ultrasound image and an ultrasound image acquisition and save mode for automatically saving the ultrasound image, according to some embodiments;

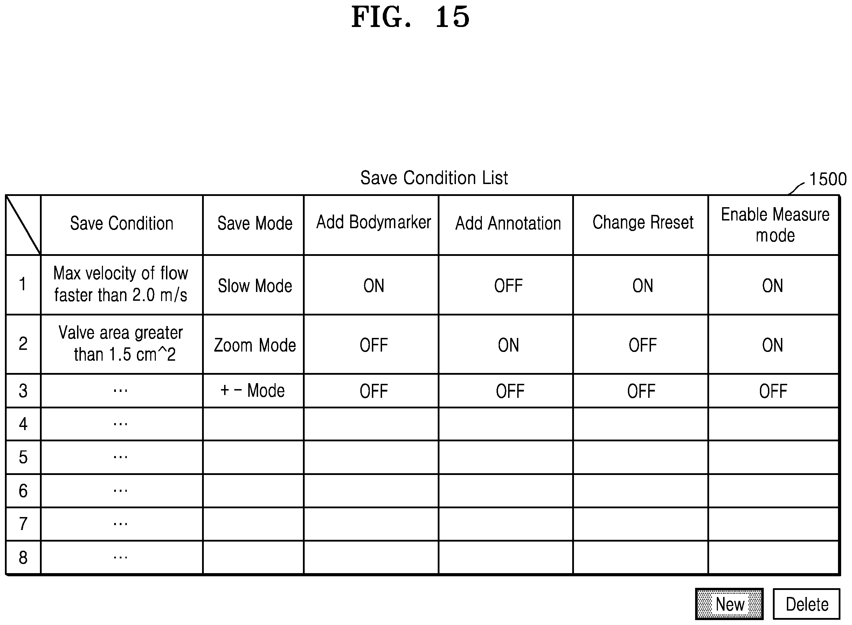

FIG. 15 illustrates an example in which an ultrasound imaging apparatus stores settings for automatically saving an ultrasound image as a list, according to some embodiments;
FIG. 16 illustrates an example in which an ultrasound imaging apparatus changes in real-time a condition for automatically saving an ultrasound image and an ultrasound image acquisition and save mode for automatically saving the ultrasound image, according to some embodiments; and
FIG. 17 illustrates an example in which an ultrasound imaging apparatus plays back an automatically saved cine image, according to some embodiments.

### DETAILED DESCRIPTION

Certain exemplary embodiments are described in greater detail below with reference to the accompanying drawings.

In the following description, the same drawing reference numerals are used for the same elements even in different drawings. The matters defined in the description, such as detailed construction and elements, are provided to assist in a comprehensive understanding of exemplary embodiments. Thus, it is apparent that exemplary embodiments can be carried out without those specifically defined matters. Also, well-known functions or constructions are not described in detail since they would obscure exemplary embodiments with unnecessary detail.

Terms such as "part" and "portion" used herein denote those that may be embodied by software or hardware. According to exemplary embodiments, a plurality of parts or portions may be embodied by a single unit or element, or a single part or portion may include a plurality of elements. Expressions such as "at least one of," when preceding a list of elements, modify the entire list of elements and do not modify the individual elements of the list.

In exemplary embodiments, an image may include any medical image acquired by various medical imaging apparatuses such as a magnetic resonance imaging (MRI) apparatus, a computed tomography (CT) apparatus, an ultrasound imaging apparatus, or an X-ray apparatus.

Also, in the present specification, an "object", which is a thing to be imaged, may include a human, an animal, or a part thereof. For example, an object may include a part of a human, that is, an organ or a tissue, or a phantom.

Throughout the specification, an ultrasound image refers to an image of an object processed based on ultrasound signals transmitted to the object and reflected therefrom.

Hereinafter, embodiments of the disclosure will be described in detail with reference to the accompanying drawings.

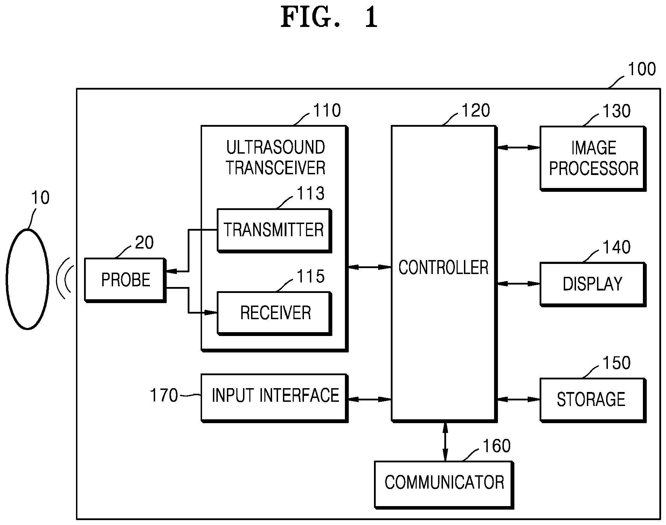

FIG. 1 is a block diagram illustrating a configuration of an ultrasound imaging apparatus 100, i.e., a diagnostic apparatus, according to an exemplary embodiment.

Referring to FIG. 1, the ultrasound imaging apparatus 100 may include a probe 20, an ultrasound transceiver 110, a controller 120, an image processor 130, one or more displays 140, a storage 150, e.g., a memory, a communicator 160, i.e., a communication device or an interface, and an input interface 170.

The ultrasound imaging apparatus 100 may be of a cart-type or a portable-type ultrasound imaging apparatus, which is portable, moveable, mobile, or hand-held. Examples of the portable-type ultrasound imaging apparatus may include a smart phone, a laptop computer, a personal digital assistant (PDA), and a tablet personal computer (PC), each of which may include a probe and a software application, but embodiments are not limited thereto.

The probe 20 may include a plurality of transducers. The plurality of transducers may transmit ultrasound signals to an object 10 in response to transmitting signals received by the probe 20, from a transmitter 113. The plurality of transducers may receive ultrasound signals reflected from the object 10 to generate reception signals. In addition, the probe 20 and the ultrasound imaging apparatus 100 may be formed in one body (e.g., disposed in a single housing), or the probe 20 and the ultrasound imaging apparatus 100 may be formed separately (e.g., disposed separately in separate housings) but linked wirelessly or via wires. In addition, the ultrasound imaging apparatus 100 may include one or more probes 20 according to embodiments.

The controller 120 may control the transmitter 113 for the transmitter 113 to generate transmitting signals to be applied to each of the plurality of transducers based on a position and a focal point of the plurality of transducers included in the probe 20.

The controller 120 may control the ultrasound receiver 115 to generate ultrasound data by converting reception signals received from the probe 20 from analogue to digital signals and summing the reception signals converted into digital form, based on a position and a focal point of the plurality of transducers.

The image processor 130 may generate an ultrasound image by using ultrasound data generated from the ultrasound receiver 115.

The display 140 may display a generated ultrasound image and various pieces of information processed by the ultrasound imaging apparatus 100. The ultrasound imaging apparatus 100 may include two or more displays 140 according to the present exemplary embodiment. The display 140 may include a touch screen in combination with a touch panel.

The controller 120 may control the operations of the ultrasound imaging apparatus 100 and flow of signals between the internal elements of the ultrasound imaging apparatus 100. The controller 120 may include a memory for storing a program or data to perform functions of the ultrasound imaging apparatus 100 and a processor and/or a microprocessor (not shown) for processing the program or data. For example, the controller 120 may control the operation of the ultrasound imaging apparatus 100 by receiving a control signal from the input interface 170 or an external apparatus.

The ultrasound imaging apparatus 100 may include the communicator 160 and may be connected to external apparatuses, for example, servers, medical apparatuses, and portable devices such as smart phones, tablet personal computers (PCs), wearable devices, etc., via the communicator 160.

The communicator 160 may include at least one element capable of communicating with the external apparatuses. For example, the communicator 160 may include at least one among a short-range communication module, a wired communication module, and a wireless communication module.

The communicator 160 may receive a control signal and data from an external apparatus.

The storage 150 may store various data or programs for driving and controlling the ultrasound imaging apparatus 100, input and/or output ultrasound data, ultrasound images, applications, etc.

The input interface 170 may receive a user's input to control the ultrasound imaging apparatus 100 and may include a keyboard, button, keypad, mouse, trackball, jog switch, knob, a touchpad, a touch screen, a microphone, a motion input means, a biometrics input means, etc. For example, the user's input may include inputs for manipulating buttons, keypads, mice, trackballs, jog switches, or knobs, inputs for touching a touchpad or a touch screen, a voice input, a motion input, and a bioinformation input, for example, iris recognition or fingerprint recognition, but an exemplary embodiment is not limited thereto.

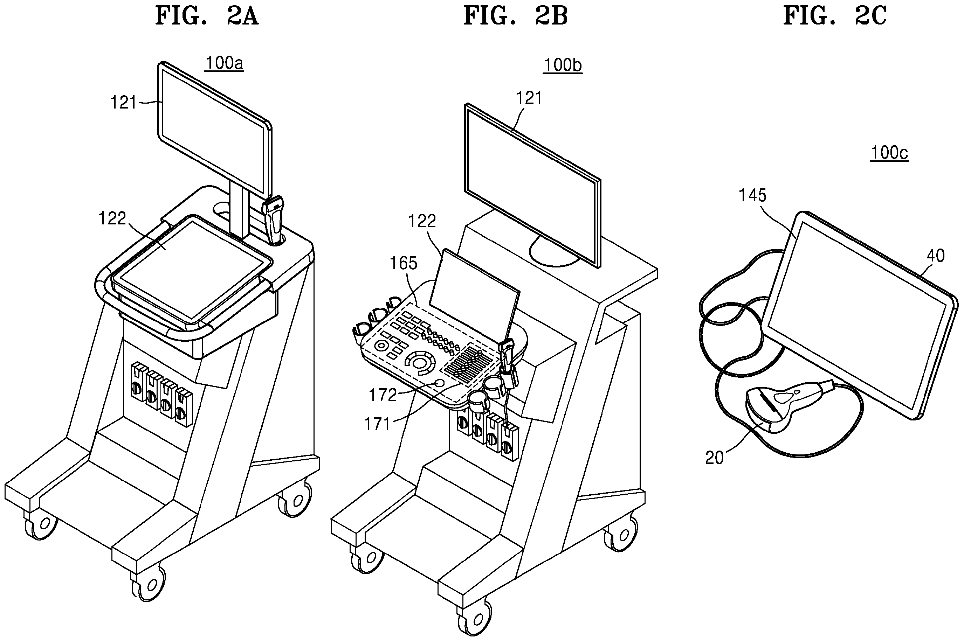

An example of the ultrasound imaging apparatus 100 according to the present exemplary embodiment is described below with reference to FIGS. 2A, 2B, and 2C.

FIGS. 2A, 2B, and 2C are diagrams illustrating ultrasound imaging apparatus according to an exemplary embodiment.

Referring to FIGS. 2A and 2B, the ultrasound imaging apparatus 100 may include a main display 121 and a sub-display 122. At least one among the main display 121 and the sub-display 122 may include a touch screen. The main display 121 and the sub-display 122 may display ultrasound images and/or various pieces of information processed by the ultrasound imaging apparatus 100. The main display 121 and the sub-display 122 may provide graphical user interfaces (GUI), thereby receiving user's inputs of data to control the ultrasound imaging apparatus 100. For example, the main display 121 may display an ultrasound image and the sub-display 122 may display a control panel to control display of the ultrasound image as a GUI. The sub-display 122 may receive an input of data to control the display of an image through the control panel displayed as a GUI. The ultrasound imaging apparatus 100 may control the display of the ultrasound image on the main display 121 by using the input control data.

Referring to FIG. 2B, the ultrasound imaging apparatus 100 may include a control panel 165. The control panel 165 may include buttons, trackballs, jog switches, or knobs, and may receive data to control the ultrasound imaging apparatus 100 from the user. For example, the control panel 165 may include a time gain compensation (TGC) button 171 and a freeze button 172. The TGC button 171 is to set a TGC value for each depth of an ultrasound image. Also, when an input of the freeze button 172 is detected during scanning an ultrasound image, the ultrasound imaging apparatus 100 may keep displaying a frame image at that time point.

The buttons, trackballs, jog switches, and knobs included in the control panel 165 may be provided as a GUI to the main display 121 or the sub-display 122.

Referring to FIG. 2C, the ultrasound imaging apparatus 100 may include a portable device. Examples of the portable ultrasound imaging apparatus may include smart phones including probes and applications, laptop computers, personal digital assistants (PDAs), or tablet PCs, but an exemplary embodiment is not limited thereto.

The ultrasound imaging apparatus 100 may include the probe 20 and a main body 40. The probe 20 may be connected to one side of the main body 40 by wire or wirelessly. The main body 40 may include a touch screen 145. The touch screen 145 may display an ultrasound image, various pieces of information processed by the ultrasound imaging apparatus 100, and a GUI.

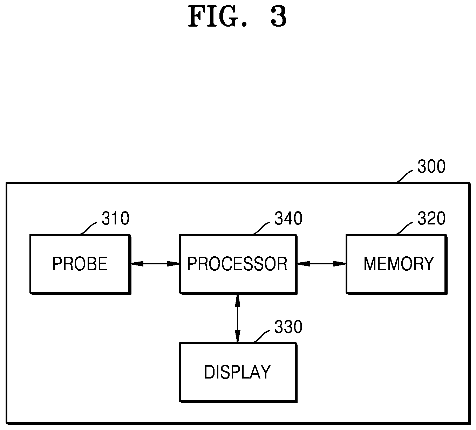

FIG. 3 illustrates a structure of an ultrasound imaging apparatus 300 according to some embodiments.

According to some embodiments, the ultrasound imaging apparatus 300 may include a probe 310, a memory 320, and a processor 340.

The ultrasound imaging apparatus 300 may be implemented in the form of an ultrasound imaging system, a general-purpose computer, a portable terminal, a kiosk, or the like. For example, the ultrasound imaging system may be implemented as the ultrasound imaging apparatus 100 of FIG. 1 or the ultrasound imaging apparatus 100a or 100b of FIG. 2.

The probe 310 may acquire ultrasound image data for generating an ultrasound image of an object. For example, the probe 310 may transmit ultrasound signals to an object and receive echo signals corresponding to the transmitted ultrasound signals. The received echo signals may be ultrasound image data used to generate an ultrasound image of the object. According to some embodiments, the probe 310 may correspond to the probe 20 described with reference to FIG. 1.

The memory 320 may store an ultrasound image. The memory 320 may also store settings for automatically saving an ultrasound image, which include a setting for a condition for automatically saving an ultrasound image, a setting for an ultrasound image acquisition and save mode, etc.

The memory 320 may be composed of a volatile or non-volatile memory. According to some embodiments, the memory 320 may correspond to the storage 150 described with reference to FIG. 1.

According to an embodiment, the ultrasound imaging apparatus 300 may further include a display 330. Although FIG. 3 shows the embodiment in which the ultrasound imaging apparatus 300 includes the display 330, the ultrasound imaging apparatus 300 may not include the display 330. For example, the ultrasound imaging apparatus 300 may generate an image to be displayed on a display, transmit the image to an external device, and output the image via a display included in the external device.

The display 330 may display an ultrasound image. When the ultrasound image is a cine image, the display 330 may play back the cine image at a specific frame rate. For example, the display 330 may play back an ultrasound cine image at a frame rate at which the ultrasound cine image was acquired (hereinafter, referred to as an 'acquisition frame rate') or at a frame rate less or greater than the acquisition frame rate. According to some embodiments, the display 330 may correspond to the display 140 described with reference to FIG. 1.

The processor 340 may control all operations of the ultrasound imaging apparatus 300 and process data. The processor 340 may include one or more processors. According to some embodiments, the processor 340 may include one or a plurality of processors for performing operations of controlling the probe 310 and processing ultrasound signals. According to some embodiments, the processor 340 may correspond to the image processor 130 of FIG. 1 or a combination of the image processor 130 and the controller 120.

According to some embodiments, the processor 340 sets a condition for automatically saving an ultrasound image of an object. The processor 340 sets, as a condition for automatically saving an ultrasound image of an object, a case where a velocity of blood flow included in a region of interest (ROI) in a real-time ultrasound image of the object is greater than a specific value. Additional condition are cases where an area of a valve orifice in an ROI in a real-time ultrasound image of the object is greater than a specific value, or where a size of a regurgitant jet area in an ROI in a real-time ultrasound image of the object is greater than a specific value.

According to some embodiments, the processor 340 sets, according to the condition for automatically saving an ultrasound image of an object, an ultrasound image acquisition and save mode for automatically acquiring and saving the ultrasound image. The processor 340 may set whether to acquire the ultrasound image of the object at a predetermined frame rate or higher and save the ultrasound image of the object, whether to obtain an ultrasound image of an object and acquire and save an enlarged version of an image of a region corresponding to an ROI in the obtained ultrasound image, and a time period during which the ultrasound image of the object to be automatically saved is acquired, etc.

According to some embodiments, the processor 340 may acquire and save an ultrasound image of an object based on an ultrasound image acquisition and save mode. In this case, acquisition of an ultrasound image of an object may mean generation of the ultrasound image based on ultrasound image data acquired via the probe 310. Generation of an ultrasound image may mean generation of both an ultrasound image to be displayed on the display 330 and an ultrasound image file to be stored in the memory 320. Thus, acquisition of an ultrasound image of an object may include generation of an ultrasound image to be displayed and generation of an ultrasound image file to be stored in the memory 320.

According to some embodiments, when a real-time ultrasound image of an object satisfies a condition for automatically saving an ultrasound image of the object, the processor 340 may automatically acquire and save an ultrasound image of the object based on a set ultrasound image acquisition and save mode. According to settings of the ultrasound image acquisition and save mode, the processor 340 may acquire the ultrasound image of the object at a predetermined frame rate or higher and save the acquired ultrasound image of the object, or may obtain an ultrasound image of an object and then acquire and save an enlarged version of an image of a portion corresponding to an ROI in the obtained ultrasound image.

According to some embodiments, the processor 340 may acquire an ultrasound image of an object during a time set based on an ultrasound image acquisition and save mode and may control the memory 320 to store the ultrasound image acquired during the set time as a cine image.

According to some embodiments, when the stored ultrasound image is an ultrasound image acquired and saved at the predetermined frame rate or higher, the processor 340 may play back the stored ultrasound image in a slow mode. In this case, the slow mode may be a mode in which the stored ultrasound image is played back at a speed less than that at which the ultrasound image has been acquired, as described in more detail below. According to some embodiments, the processor 340 may add at least one or a combination of an annotation and a body marker to the acquired ultrasound image. The processor 340 may control the memory 320 to store the ultrasound image to which at least one or a combination of the body marker and the annotation is added.

According to some embodiments, as an ultrasound image is automatically acquired and saved, the processor 340 may automatically execute functions related to the saved ultrasound image. The functions related to the saved ultrasound image may include a diagnostic function with respect to an object in the saved ultrasound image, a measurement function with respect to the saved ultrasound image, etc.

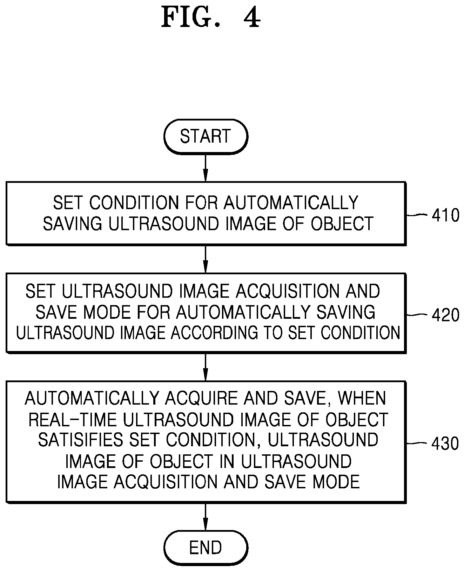

FIG. 4 is a flowchart of a method, performed by the ultrasound imaging apparatus 300, of automatically saving an ultrasound image, according to some embodiments.

Referring to FIG. 4, the ultrasound imaging apparatus 300 may set a condition for automatically saving an ultrasound image of an object (operation 410).

According to some embodiments, the ultrasound imaging apparatus 300 may set a case where a particular lesion is seen on a real-time ultrasound image of an object as a condition for automatically saving an ultrasound image of the object.

For example, the ultrasound imaging apparatus 300 may set a case where reversal of cardiac blood flow is seen on a real-time cardiac ultrasound image of an object as a condition for automatically saving an ultrasound image of the object.

According to the invention, the ultrasound imaging apparatus 300 sets a case where a velocity of blood flow included in an ROI in a real-time ultrasound image of an object is greater than a specific value as a condition for automatically saving an ultrasound image of the object.

For example, the ultrasound imaging apparatus 300 may set a case where a velocity of blood flow measured in a sample gate set in the ROI in the real-time ultrasound image of the object is greater than a specific value as a condition for automatically saving an ultrasound image of the object.

According to some embodiments, when a case where a velocity of blood flow included in an ROI in a real-time ultrasound image of an object is greater than a specific value is set as a condition for automatically saving an ultrasound image of the object, the ultrasound imaging apparatus 300 may set the specific value as a reference for determination.

For example, the ultrasound imaging apparatus 300 may set a velocity (e.g., 2 m/s) of blood flow that is used as a reference in determining that reversal of blood flow occurs in the heart to a specific value that is a reference value for determining whether to automatically save an ultrasound image.

According to some embodiments, the ultrasound imaging apparatus 300 may set a case where an area of a valve orifice included in an ROI in a real-time ultrasound image of an object is greater than a specific value as a condition for automatically saving an ultrasound image of the object.

According to some embodiments, when a case where an area of a valve orifice in an ROI in a real-time ultrasound image of an object is greater than a specific value is set as a condition for automatically saving an ultrasound image of the object, the ultrasound imaging apparatus 300 may set the specific value as a reference for determination.

For example, the ultrasound imaging apparatus 300 may set an area of a mitral valve orifice when a mitral valve opens to its maximum extent to a specific value that is a reference value for determining whether to automatically save an ultrasound image. In this case, the area of the mitral valve orifice is used as a reference for determining the degree of mitral stenosis in the heart.

According to some embodiments, the ultrasound imaging apparatus 300 may set a case where a size of a regurgitant jet area included in an ROI in a real-time ultrasound image of an object is greater than a specific value as a condition for automatically saving an ultrasound image of the object. In this case, the regurgitant jet area may be an area in which reversal of blood flow occurs. The regurgitant jet area may be detected via determination of a direction of blood flow in a color Doppler ultrasound image.

According to some embodiments, when a case where a size of a regurgitant jet area in an ROI in a real-time ultrasound image of an object is greater than a specific value is set as a condition for automatically saving an ultrasound image of the object, the ultrasound imaging apparatus 300 may set the specific value as a reference for determination.

For example, the ultrasound imaging apparatus 300 may set a size of a regurgitant jet area, which is used as a reference for determining that reversal of blood flow occurs in the heart, to a specific value that is a reference value for determining whether to automatically save an ultrasound image.

According to some embodiments, the ultrasound imaging apparatus 300 may set, as a condition for automatically saving an ultrasound image, a case where a velocity of blood flow included in a region of interest (ROI) in a real-time ultrasound image of the object is greater than a specific value, or a combination of this case with one or both of the cases where an area of a valve orifice in an ROI in a real-time ultrasound image of the object is greater than a specific value, and where a size of a regurgitant jet area in an ROI in a real-time ultrasound image of the object is greater than a specific value.

According to some embodiments, the ultrasound imaging apparatus 300 may receive from a user an input of setting a condition for automatically saving an ultrasound image of an object. The ultrasound imaging apparatus 300 may set the condition for automatically saving the ultrasound image of the object based on the received input.

The ultrasound imaging apparatus 300 may set an ultrasound image acquisition and save mode for automatically acquiring and saving the ultrasound image of the object according to the set condition (operation 420).

According to some embodiments, settings of an ultrasound image acquisition and save mode for automatically acquiring and saving an ultrasound image of an object may include a setting of a time period during which the ultrasound image of the object is acquired, a setting of a frame rate at which the ultrasound image is acquired, a setting with regard to whether to acquire and save an enlarged version of the ultrasound image, etc.

According to some embodiments, the ultrasound imaging apparatus 300 may set a time period needed to acquire an ultrasound image of an object to be automatically saved. In this case, the time period needed to acquire the ultrasound image of the object may mean a length of time during which the ultrasound image of the object to be automatically saved is acquired.

According to some embodiments, the ultrasound imaging apparatus 300 may preset the time period needed to acquire the ultrasound image of the object to be automatically saved (hereinafter referred to as an 'ultrasound image acquisition time period') to a specific value. For example, when a real-time ultrasound image of the object satisfies a condition for automatically saving an ultrasound image of the object, the ultrasound imaging apparatus 300 may preset the ultrasound image acquisition time period to automatically save an ultrasound image of the object that is obtained during a specific time period (e.g., 5s) from a time point when the real-time ultrasound image is acquired.

Furthermore, when a real-time ultrasound image of the object satisfies a condition for automatically saving an ultrasound image of the object, the ultrasound imaging apparatus 300 may preset the ultrasound image acquisition time period to automatically save an ultrasound image of the object that has been obtained during a specific time period before acquisition of the real-time ultrasound image or after the acquisition of the real-time ultrasound image. In this case, the ultrasound image obtained during the specific time period before the acquisition of the real-time ultrasound image may be temporarily stored in a buffer of the ultrasound imaging apparatus 300.

According to another embodiment, the ultrasound imaging apparatus 300 may automatically set an ultrasound image acquisition time period according to a condition for automatically saving an ultrasound image of an object. For example, the ultrasound imaging apparatus 300 may set an ultrasound image acquisition time period for each condition for automatically saving an ultrasound image, i.e., each auto save condition based on a velocity of blood flow, an area of a valve orifice, or a size of a regurgitant jet area.

For example, when an ultrasound image is automatically saved based on a velocity of blood flow, the ultrasound imaging apparatus 300 may set an ultrasound image acquisition time period to automatically save an ultrasound image of an object that is obtained during a specific time period that occurs before or after a time point when a real-time ultrasound image satisfying a condition of the velocity of blood flow is acquired.

According to another embodiment, an ultrasound image acquisition time period may be set based on a user input. For example, the ultrasound imaging apparatus 300 may set an ultrasound image acquisition time period based on a user input of setting the ultrasound image acquisition time period to automatically save an ultrasound image of an object that is obtained within a specific time period (e.g., 5s) from acquisition of a real-time ultrasound image of the object satisfying a condition for automatically saving an ultrasound image. According to some embodiments, when an ultrasound image of an object is automatically acquired and saved according to a condition for automatically saving the ultrasound image, the ultrasound imaging apparatus 300 may set a frame rate at which the ultrasound image of the object to be automatically saved is acquired.

A frame rate refers to a ratio of a speed at which consecutive images are acquired, captured, or played back and may mean the number of images acquired or played back per unit time. A frame rate may be the same as a frame speed or frames per unit time. The frame rate may be expressed in frames per second (fps).

According to some embodiments, when an ultrasound image of an object is automatically acquired and saved according to a condition for automatically saving the ultrasound image, the ultrasound imaging apparatus 300 may set whether to acquire and the ultrasound image at a predetermined frame rate or higher and save the ultrasound image. For example, the ultrasound imaging apparatus 300 may set, based on a user input, whether to acquire an ultrasound image of an object at a predetermined frame rate or higher and to automatically save the acquired ultrasound image of the object. Furthermore, when the condition for automatically saving an ultrasound image is set, the ultrasound imaging apparatus 300 may be automatically set to acquire the ultrasound image of the object to be automatically saved at the predetermined frame rate or higher and save the ultrasound image of the object.

According to some embodiments, when an ultrasound image of an object is automatically acquired and saved, the ultrasound imaging apparatus 300 may set whether to acquire and save the ultrasound image of the object at a high frame rate. In this case, a high frame rate may be a frame rate that is greater than or equal to a standard frame rate (or a default frame rate) at which the ultrasound imaging apparatus 300 acquires an ultrasound image. The standard frame rate may be a frame rate at which the ultrasound imaging apparatus 300 acquires an ultrasound image in real-time.

According to some embodiments, the ultrasound imaging apparatus 300 may store an ultrasound image to be automatically saved at a high frame rate and play back the ultrasound image in a slow mode. In this case, the high frame rate at which the ultrasound image is stored may be determined to be greater than or equal to a minimum frame rate for supporting a slow mode. The slow mode is a mode in which the ultrasound image is played back at a speed lower than that at which the ultrasound image has been captured during acquisition. For example, when an image captured for one (1) minute is played back for 1 minute in a normal mode, an image captured for thirty (30) seconds may be played back for 1 minute in a slow mode. According to an embodiment, by capturing an ultrasound image to be automatically saved at a high frame rate and playing back the automatically saved ultrasound image in a slow mode, it is possible to support the slow mode while preserving a set playback frame rate. For example, according to an embodiment, when an ultrasound image is played back at 60 fps, the ultrasound image may be saved at 120 fps and played back at 60 fps at 0.5 × speed.

According to some embodiments, the ultrasound imaging apparatus 300 may preset a value corresponding to a high frame rate to a particular value. According to another embodiment, the ultrasound imaging apparatus 300 may automatically set a value corresponding to a high frame rate according to a condition for automatically saving an ultrasound image of an object. For example, the ultrasound imaging apparatus 300 may set a high frame rate for each condition for automatically saving an ultrasound image of an object, i.e., each auto save condition based on a velocity of blood flow, an area of a valve orifice, or a size of a regurgitant jet area. According to another embodiment, a high frame rate may be set based on a user input. For example, the user input may include an input of setting a high frame rate, an input of setting a playback speed at a slow mode, etc.

According to the invention the ultrasound imaging apparatus 300 obtains an ultrasound image of an object and set whether to acquire and save an enlarged version of an image of a portion corresponding to an ROI in the obtained ultrasound image. For example, the ultrasound imaging apparatus 300 may set, based on a user input, whether to acquire and save an enlarged version of an image of a portion corresponding to an ROI in an obtained ultrasound image. Furthermore, as the condition for automatically saving an ultrasound image is set, the ultrasound imaging apparatus 300 may be automatically set to automatically acquire and save the enlarged version of the image of the portion corresponding to the ROI in the obtained ultrasound image. When an ultrasound image of an object is acquired and saved according to a condition for automatically saving the ultrasound image of the object, the ultrasound imaging apparatus may automatically store the acquired ultrasound image as well as an enlarged version of an image of a region corresponding to an ROI in the acquired ultrasound image.

According to some embodiments, a setting of an ultrasound image acquisition time period, a setting of an ultrasound image acquisition frame rate, a setting with regard to whether to acquire and save an enlarged version of the ultrasound image, etc., which are included in settings of an ultrasound image acquisition and save mode, may be set independently from one another. For example, when a real-time ultrasound image satisfies a condition for automatically saving an ultrasound image, the ultrasound imaging apparatus 300 may set an ultrasound image acquisition and save mode to automatically acquire an ultrasound image at a predetermined frame rate or higher and save the ultrasound image while simultaneously acquiring and saving an enlarged version of an image of a portion corresponding to an ROI.

When a real-time ultrasound image of the object satisfies the set condition, the ultrasound imaging apparatus 300 may automatically acquire and save the ultrasound image of the object based on the ultrasound image acquisition and save mode (operation 430).

According to some embodiments, the ultrasound imaging apparatus 300 may determine whether a real-time ultrasound image of the object satisfies the set condition for automatically saving an ultrasound image.

For example, when a case where a particular lesion is seen on a real-time ultrasound image of an object is set as a condition for automatically saving an ultrasound image of the object, the ultrasound imaging apparatus 300 may determine whether the particular lesion is seen on the real-time ultrasound image. For example, the ultrasound imaging apparatus 300 may determine whether reversal of cardiac blood flow is seen on a real-time cardiac ultrasound image of an object. The ultrasound imaging apparatus 300 may determine or automatically determine whether the particular lesion is seen on the real-time ultrasound image of the object, based on a user input or a prestored determination method. However, a method by which the ultrasound imaging apparatus 300 determines whether a particular lesion appears is not limited to the above-described method based on the user input, etc.

Furthermore, when a case where a velocity of blood flow included in an ROI in a real-time ultrasound image of an object is greater than a specific value is set as a condition for automatically saving an ultrasound image of the object, the ultrasound imaging apparatus 300 may determine whether the velocity of blood flow in the ROI is greater than the specific value.

For example, the ultrasound imaging apparatus 300 may measure a velocity of blood flow in a sample gate set in an ROI in a real-time ultrasound image of an object. The ultrasound imaging apparatus 300 may also determine whether the velocity of blood flow measured in the sample gate is greater than a specific value. The ultrasound imaging apparatus 300 may determine whether the velocity of blood flow in the ROI is greater than the specific value based Doppler data acquired at the sample gate.

Furthermore, when a real-time ultrasound image of an object is a color Doppler image, the ultrasound imaging apparatus 300 may determine whether a color representing a higher velocity than a specific velocity is seen in an ROI in the real-time ultrasound image. When the color representing a higher velocity than the specific velocity is seen in the ROI in the real-time ultrasound image, the ultrasound imaging apparatus 300 may determine that a velocity of blood flow included in the ROI in the real-time ultrasound image is greater than the specific velocity.

Furthermore, the ultrasound imaging apparatus 300 may determine whether an area of a valve orifice in an ROI in a real-time ultrasound image of an object is greater than a specific value. The ultrasound imaging apparatus 300 may detect opening and closing of a valve included in the ROI in the real-time ultrasound image. For example, the ultrasound imaging apparatus 300 may detect opening and closing of a valve included in the ROI in the real-time ultrasound image of the object. For example, the ultrasound imaging apparatus 300 may detect opening and closing of a valve by using a method of detecting motion of tissue in a particular region of the object in a real-time ultrasound image. When opening of the valve is detected, the ultrasound imaging apparatus 300 may detect a valve orifice by recognizing segmentation of the object. The ultrasound imaging apparatus 300 may measure an area of the detected valve orifice by using a method of measuring a size of a region in an ultrasound image.

Furthermore, the ultrasound imaging apparatus 300 may detect the valve orifice in the real-time ultrasound image based on a user input and measure an area of the valve orifice. Furthermore, the ultrasound imaging apparatus 300 may automatically detect the valve orifice and measure the area of the valve orifice based on prestored valve orifice detection and valve orifice area measurement methods.

Furthermore, the ultrasound imaging apparatus 300 may determine whether a size of a regurgitant jet area in a real-time ultrasound image of an object is greater than a specific value. For example, the ultrasound imaging apparatus 300 may detect whether the regurgitant jet area appears on the real-time ultrasound image. The ultrasound imaging apparatus 300 may detect an area in which regurgitant jet appears by comparing a blood flow velocity, i.e., a flow direction and a velocity magnitude, on a frame-by-frame basis. The ultrasound imaging apparatus 300 may measure a size of the detected regurgitant jet area by using a method of measuring a size of a region in an ultrasound image. The ultrasound imaging apparatus 300 may also determine whether the measured size is greater than a specific value.

Furthermore, the ultrasound imaging apparatus 300 may detect, based on a user input, a regurgitant jet area in a real-time ultrasound image and measure a size of the regurgitant jet area. Furthermore, the ultrasound imaging apparatus 300 may automatically detect a regurgitant jet area and measure a size of the regurgitant jet area based on prestored regurgitant jet area detection and size measurement methods.

According to some embodiments, when it is determined that a real-time ultrasound image of an object satisfies a set condition for automatically saving an ultrasound image of the object, the ultrasound imaging apparatus 300 may automatically acquire and save an ultrasound image of the object based on an ultrasound image acquisition and save mode.

As described above, the ultrasound image acquisition and save mode may include a setting of an ultrasound image acquisition time period, a setting of an ultrasound image acquisition frame rate, a setting with regard to whether to acquire and save an enlarged version of an ultrasound image, etc.

According to some embodiments, when it is determined that a real-time ultrasound image of an object satisfies a set condition for automatically saving an ultrasound image, the ultrasound imaging apparatus 300 may automatically acquire and save the real-time ultrasound image of the object determined to satisfy the set condition.

According to some embodiments, the ultrasound imaging apparatus 300 may obtain, based on a setting of an ultrasound image acquisition time period included in an ultrasound image acquisition and save mode, an ultrasound image of an object to be saved during the set ultrasound image acquisition time period.

For example, the ultrasound imaging apparatus 300 may obtain, based on a setting of an ultrasound image acquisition time period, an ultrasound image of the object during a specific time period from acquisition of a real-time ultrasound image of the object which satisfies a condition for automatically saving an ultrasound image.

Furthermore, the ultrasound imaging apparatus 300 may obtain, based on a setting of an ultrasound image acquisition time period, an ultrasound image of an object during a specific time period before or after a time point when a real-time ultrasound image of the object which satisfies a condition for automatically saving an ultrasound image is acquired. The ultrasound image of the object over the specific time period before acquisition of the real-time ultrasound image of the object may be a preacquired image or may be temporarily stored after acquisition.

According to some embodiments, the ultrasound imaging apparatus 300 may acquire, based on a setting of an ultrasound image acquisition frame rate included in an ultrasound image acquisition and save mode, an ultrasound image of an object at the set frame rate. In this case, the setting of the ultrasound image acquisition frame rate may include a setting with regard to whether to acquire the ultrasound image of the object at a predetermined frame rate or higher and save the ultrasound image of the object.

For example, the ultrasound imaging apparatus 300 may obtain, based on a setting of an ultrasound image acquisition frame rate, an ultrasound image of an object to be saved at a predetermined frame rate or higher.

Furthermore, the ultrasound imaging apparatus 300 may obtain, based on a setting of an ultrasound image acquisition frame rate, an ultrasound image of an object at a high frame rate. As described above, a high frame rate may be a frame rate that is greater than or equal to a default frame rate at which the ultrasound imaging apparatus 300 acquires an ultrasound image.

According to some embodiments, the ultrasound imaging apparatus 300 may obtain an ultrasound image of an object at a predetermined frame rate or higher by focusing a beam on a region of the object.

When the ultrasound imaging apparatus 300 transmits a beam to the entire area of an object to acquire an ultrasound image of the object at a predetermined frame rate or higher (e.g., at a high frame rate), the acquired ultrasound image may have a low image quality. Thus, to acquire an ultrasound image at the predetermined frame rate or higher without compromising image quality, the ultrasound imaging apparatus 300 may need to adjust the number and depth of beam lines or scan lines for obtaining an ultrasound image.

The ultrasound imaging apparatus 300 may reduce the number of beam lines and a depth for a beam line by focusing a beam on a region of the object. For example, the ultrasound imaging apparatus 300 may focus a beam only on a region of the object or on both the region of the object and its surrounding area.

The ultrasound imaging apparatus 300 may focus a beam on a region of the object to acquire an ultrasound image at a predetermined frame rate or higher. Thus, the ultrasound imaging apparatus 300 may obtain the ultrasound image at the predetermined frame rate or higher with a little image quality loss in the region where the beam is focused. For example, the ultrasound imaging apparatus 300 may focus a beam on an ROI of the object to obtain an ultrasound image at a predetermined frame rate or higher with a little image quality loss. Furthermore, the ultrasound imaging apparatus 300 may focus a beam on a region of blood flow in an object to obtain a blood flow ultrasound image at a predetermined frame rate or higher with a little image quality loss.

According to the invention the ultrasound imaging apparatus 300 automatically stores, based on a setting with respect to whether to acquire and save an enlarged version of an image of a portion corresponding to an ROI in an obtained ultrasound image, the enlarged version of the image of the portion corresponding to the ROI (or an enlarged version of an image of the ROI).

According to the invention, the ultrasound imaging apparatus 300 focuses a beam on a region of an object to obtain an enlarged version of an image of a portion corresponding to the region of the object. Alternatively, not in accordance with the present invention, the ultrasound imaging apparatus 300 may focus a beam on a region of an object and its surrounding area to obtain an enlarged version of an image of a portion corresponding to the region of the object and the surrounding area. For example, the ultrasound imaging apparatus 300 may focus a beam on an ROI of the object to obtain an enlarged version of an image of a portion corresponding to the ROI of the object.

The ultrasound imaging apparatus 300 focuses a beam on a region of an object to acquire an enlarged version of an image of a portion corresponding to the region of the object. Thus, the ultrasound imaging apparatus 300 may obtain the enlarged version of the image of the region where the beam is focused with a little loss in image quality. For example, the ultrasound imaging apparatus 300 may focus a beam on an ROI of an object to acquire an ultrasound image, thereby obtaining an enlarged version of an image of the ROI without significantly compromising the image quality.

According to some embodiments, based on an ultrasound image acquisition and save mode, the ultrasound imaging apparatus 300 may acquire an ultrasound image of an object at a predetermined frame rate or higher and save the ultrasound image of the object while simultaneously acquiring and saving an enlarged version of an image of a portion corresponding to an ROI in the ultrasound image. For example, the ultrasound imaging apparatus 300 may acquire and save an enlarged version of an image of a portion corresponding to an ROI in an obtained real-time ultrasound image at a predetermined frame rate or higher. By focusing a beam on the ROI of the object, the ultrasound imaging apparatus 300 may acquire and save the enlarged version of the image of the portion corresponding to the ROI at the predetermined frame rate or higher. According to some embodiments, the ultrasound imaging apparatus 300 may save as a cine image ultrasound images of an object automatically acquired based on an ultrasound image acquisition and save mode. For example, the ultrasound imaging apparatus 300 may acquire ultrasound images of an object during a preset time period based on a setting of an ultrasound image acquisition time period and save the acquired ultrasound images as a cine image.

A cine image is a series of cross-sectional images of an internal part of an object, which are arranged sequentially over time, and may be used to examine movement of the internal part of the object. In general, a cine image of the heart may be used to determine the presence of cardiac dysfunction such as a reversal of cardiac blood flow.

The ultrasound imaging apparatus 300 may store automatically acquired ultrasound images of an object as a cine image according to a sequential order in which the ultrasound images were acquired.

The ultrasound imaging apparatus 300 is capable of automatically acquiring and saving an ultrasound image according to a set condition without a user's manual input, thereby accurately and promptly acquiring and saving an ultrasound image of a region to be diagnosed without a user's manual input.

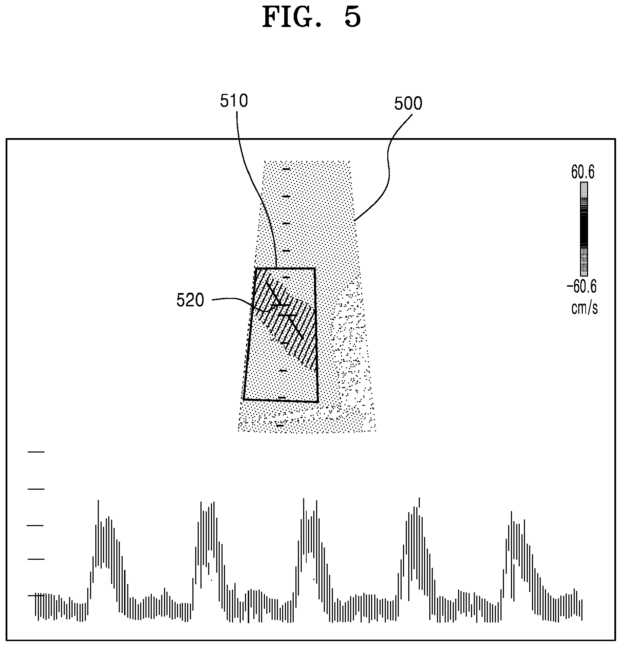

FIG. 5 illustrates an example in which the ultrasound imaging apparatus 300 automatically saves an ultrasound image when a condition for automatically saving an ultrasound image is set based on a velocity of blood flow, according to some embodiments.

FIG. 5 shows a real-time ultrasound image 500 acquired by the ultrasound imaging apparatus 300. The real-time ultrasound image 500 may be a color Doppler ultrasound image. Furthermore, a ROI 510 may be set in the real-time ultrasound image 500. A sample gate 520 for measuring a velocity of blood flow may be set in the ROI 510.

The ultrasound imaging apparatus 300 may set a case where a velocity of blood flow included in the acquired real-time ultrasound image 500 is greater than a specific value as a condition for automatically saving an ultrasound image. When the condition for automatically saving an ultrasound image is set based on the velocity of blood flow, the ultrasound imaging apparatus 300 may measure the blood flow in the real-time ultrasound image 500 and determine whether the real-time ultrasound image 500 satisfies the set condition.

For example, the ultrasound imaging apparatus 300 may measure a velocity of blood flow in the real-time ultrasound image 500 via the sample gate 520 set in the ROI 510. When the measured velocity of blood flow is greater than the specific value in the set condition, it may be determined that the real-time ultrasound image 500 satisfies the condition for automatically saving an ultrasound image. When it is determined that the real-time ultrasound image 500 satisfies the condition, the ultrasound imaging apparatus 300 may automatically acquire and save an ultrasound image of an object based on an ultrasound image acquisition and save mode for automatically acquiring and saving the ultrasound image.

While FIG. 5 shows an example in which the real-time ultrasound image 500 is a color Doppler image and a velocity of blood flow is measured using the sample gate 520, methods by which the ultrasound imaging apparatus 300 determines a velocity of blood flow in a real-time ultrasound image according to embodiments are not limited thereto.

A velocity of blood flow is clinically important in various tests. For example, the degree of arterial stenosis may be determined by measuring a velocity of blood flow in a color Doppler image during carotid ultrasound imaging. Thus, when a condition for automatically saving an ultrasound image is set based on the velocity of blood flow, an ultrasound image of a region where the arterial stenosis occurs may be automatically acquired and saved.

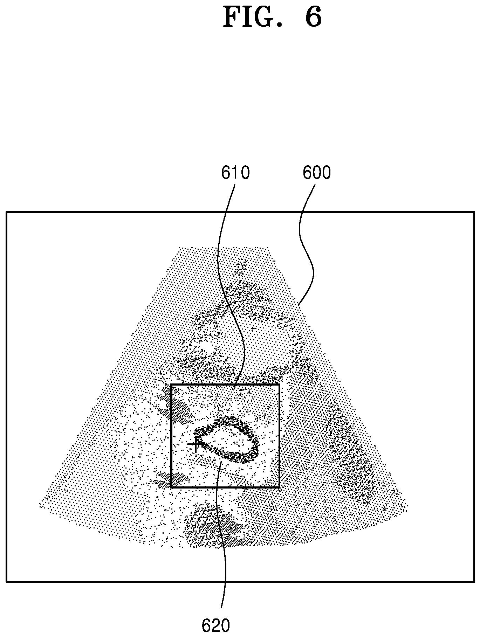

FIG. 6 illustrates an example in which the ultrasound imaging apparatus 300 automatically saves an ultrasound image when a condition for automatically saving an ultrasound image is set based on an area of a valve orifice, according to some embodiments.

FIG. 6 shows a real-time ultrasound image 600 acquired by the ultrasound imaging apparatus 300. The real-time ultrasound image 600 may be an ultrasound image of the heart of an object. Furthermore, an ROI 610 may be set in the real-time ultrasound image 600.

The ultrasound imaging apparatus 300 may set a case where an area of a valve orifice 620 included in the acquired real-time ultrasound image 600 is greater than a specific value as a condition for automatically saving an ultrasound image. When the condition for automatically saving an ultrasound image is set based on the area of the valve orifice, the ultrasound imaging apparatus 300 may measure the area of the valve orifice in the real-time ultrasound image 600 and determine whether the real-time ultrasound image 600 satisfies the set condition.

For example, the ultrasound imaging apparatus 300 may measure an area of the valve orifice 620 included in the ROI 610 in the real-time ultrasound image 600. In detail, the ultrasound imaging apparatus 300 may detect opening and closing of a valve by using a method of detecting motion of tissue in a particular region of the object. When opening of the valve is detected, the ultrasound imaging apparatus 300 may detect the valve orifice 620 by recognizing segmentation of the object. The ultrasound imaging apparatus 300 may measure an area of the detected valve orifice 620 by using a method of measuring a size of a region in an ultrasound image.

When the measured area of the valve orifice 620 is greater than the specific value in the set condition, it may be determined that the real-time ultrasound image 600 satisfies the condition for automatically saving an ultrasound image.

When it is determined that the real-time ultrasound image 600 satisfies the condition, the ultrasound imaging apparatus 300 may automatically acquire and save an ultrasound image of an object based on an ultrasound image acquisition and save mode for automatically acquiring and saving the ultrasound image.

The degree of mitral stenosis in the heart may be determined based on an area of a mitral valve orifice when a mitral valve opens to its maximum extent. When the maximum area of the mitral valve orifice is less than a specific value, it is determined that mitral stenosis occurs in the heart. When a condition for automatically saving an ultrasound image is set based on an area of a valve orifice, an ultrasound image of a region including the mitral valve orifice when the mitral valve opens may be acquired and saved. By using the saved ultrasound image, the maximum area of the mitral valve orifice may be measured, and whether mitral stenosis occurs may be determined based on the measured maximum area.

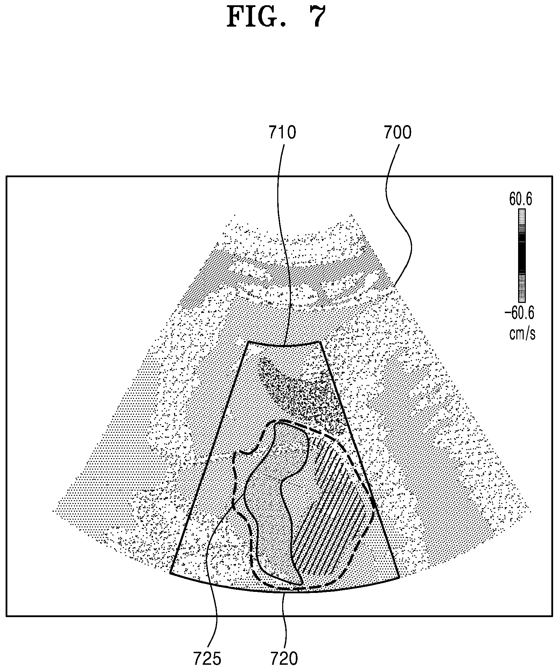

FIG. 7 illustrates an example in which the ultrasound imaging apparatus 300 automatically saves an ultrasound image when a condition for automatically saving an ultrasound image is set based on a size of a regurgitant jet area, according to some embodiments.

FIG. 7 shows a real-time ultrasound image 700 acquired by the ultrasound imaging apparatus 300. The real-time ultrasound image 700 may be a color Doppler ultrasound image of the heart of an object. Furthermore, an ROI 710 may be set in the real-time ultrasound image 700.

The ultrasound imaging apparatus 300 may set a case where a size of a regurgitant jet area 725 included in the acquired real-time ultrasound image 700 is greater than a specific value as a condition for automatically saving an ultrasound image. When the condition for automatically saving an ultrasound image is set based on the size of the regurgitant jet area 725, the ultrasound imaging apparatus 300 may measure the size of the regurgitant jet area 725 in the real-time ultrasound image 700 and determine whether the real-time ultrasound image 700 satisfies the set condition.

For example, the ultrasound imaging apparatus 300 may detect a heart region 720 included in the ROI 710 in the real-time ultrasound image 700 and then the regurgitant jet area 725 in the detected heart region 720. The ultrasound imaging apparatus 300 may detect the regurgitant jet area 725 in which regurgitant jet appears by comparing a blood flow velocity, i.e., a flow direction and a velocity magnitude, on a frame-by-frame basis. The ultrasound imaging apparatus 300 may measure a size of the detected regurgitant jet area 725 by using a method of measuring a size of a region in an ultrasound image.

When the measured size of the regurgitant jet area 725 is greater than the specific value in the set condition, it may be determined that the real-time ultrasound image 700 satisfies the condition for automatically saving an ultrasound image.

When it is determined that the real-time ultrasound image 700 satisfies the condition, the ultrasound imaging apparatus 300 may automatically acquire and save an ultrasound image of the object based on an ultrasound image acquisition and save mode for automatically acquiring and saving the ultrasound image.

One method of assessing a function of the heart may be using a still image of the largest regurgitant jet area in a captured color Doppler ultrasound image. The severity of reversal of blood flow may be determined via various measurements with respect to a still image that is a captured image of the largest regurgitant jet area, and thus, the heart function may be assessed based on a result of the determination. Thus, when a condition for automatically saving an ultrasound image is set based on a size of a regurgitant jet area, a still image captured of the largest regurgitant jet area may be automatically acquired and saved.

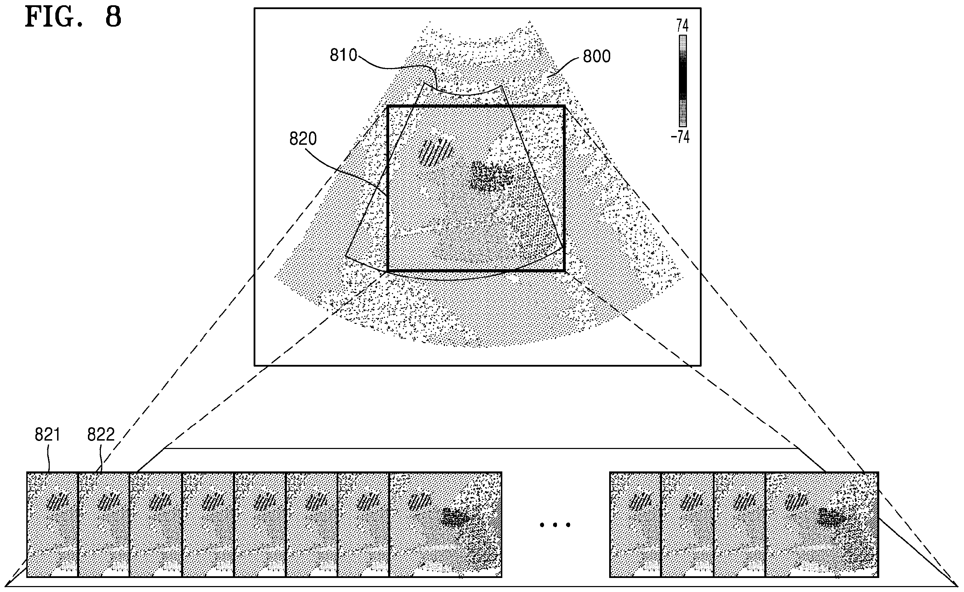

FIG. 8 illustrates an example in which the ultrasound imaging apparatus 300 automatically acquires an ultrasound image at a predetermined frame rate or higher and saves the ultrasound image, according to some embodiments.

FIG. 8 shows an example in which the ultrasound imaging apparatus 300 sets an ultrasound image acquisition and save mode to acquire an ultrasound image 800 of an object at a predetermined frame rate or higher (e. g., at a high frame rate) and save the ultrasound image 800.

FIG. 8 shows the ultrasound image 800 of the object acquired by the ultrasound imaging apparatus 300. An ROI 810 may be set in the ultrasound image 800.

When an acquired real-time ultrasound image satisfies a condition for automatically saving an ultrasound image, the ultrasound imaging apparatus 300 may automatically acquire the ultrasound image 800 of the object at the predetermined frame rate or higher and save the ultrasound image 800 during a preset time period. In this case, according to settings of the ultrasound image acquisition and save mode, the ultrasound imaging apparatus 300 may automatically acquire and save the ultrasound image 800 of the object at a high frame rate during the preset time period.

Furthermore, the ultrasound imaging apparatus 300 may focus a beam on a region of the object to obtain the ultrasound image 800 of the object at a predetermined frame rate or higher. For example, the ultrasound imaging apparatus 300 may obtain the ultrasound image 800 of the object at a predetermined frame rate or higher by focusing a beam on the ROI 810. Furthermore, the ultrasound imaging apparatus 300 may obtain the ultrasound image 800 of the object at a predetermined frame rate or higher by focusing a beam on an area 820 including a blood flow region.

The ultrasound imaging apparatus 300 may focus a beam on a region of the object to acquire an ultrasound image at a predetermined frame rate or higher. Thus, the ultrasound imaging apparatus 300 may obtain the ultrasound image with a little loss in image quality in the region where the beam is focused at the predetermined frame rate or higher.

The ultrasound imaging apparatus 300 may store acquired ultrasound images 821, 822, ... Furthermore, the ultrasound imaging apparatus 300 may store the acquired ultrasound images 821, 822, ... as a cine image according to a sequential order in which they are acquired.

The ultrasound imaging apparatus 300 may display the stored ultrasound images 821, 822, ... The ultrasound imaging apparatus 300 may also play back in a slow mode the ultrasound images that are acquired at a predetermined frame rate or higher and then stored as a cine image

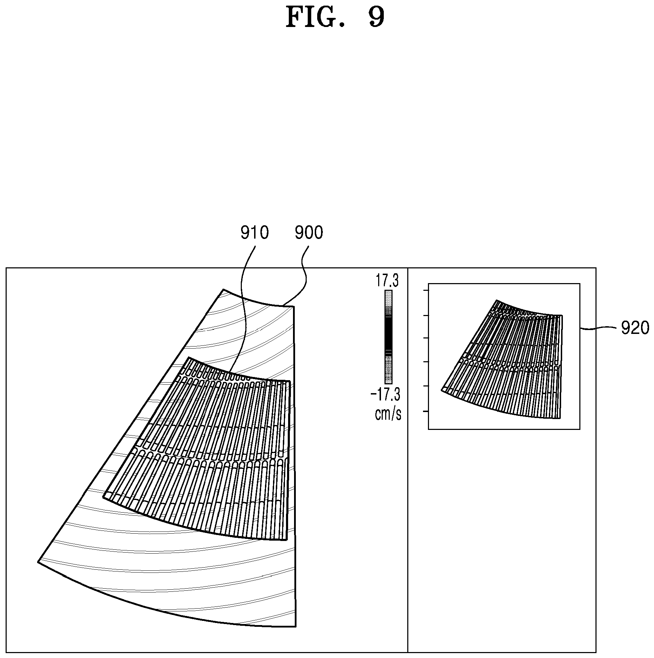

FIG. 9 illustrates an example in which the ultrasound imaging apparatus 300 automatically acquires and saves an enlarged version of an ultrasound image of a portion corresponding to a region of interest (ROI), according to some embodiments.

FIG. 9 shows an example in which the ultrasound imaging apparatus 300 obtains an ultrasound image 900 of an object and sets an ultrasound image acquisition and save mode to acquire and save an enlarged version of an image of a portion corresponding to an ROI 910 in the obtained ultrasound image 900.

FIG. 9 shows the ultrasound image 900 of the object acquired by the ultrasound imaging apparatus 300. The ROI 910 may be set in the ultrasound image 900.

Referring to FIG. 9, when an acquired real-time ultrasound image satisfies a condition for being automatically saved, the ultrasound imaging apparatus 300 obtains the ultrasound image 900 of the object and automatically saves an enlarged version 920 of an image of a portion corresponding to the ROI 910 in the obtained ultrasound image 900.

For example, the ultrasound imaging apparatus 300 focuses a beam on a region of the object to acquire the enlarged version 920 of the image of the portion corresponding to the ROI 910 in the obtained ultrasound image 900. For example, the ultrasound imaging apparatus 300 may focus a beam on the ROI 910 to acquire the enlarged version 920 of the image of the portion corresponding to the ROI 910. Because the enlarged version 920 of the image of the portion corresponding to the ROI 910 is acquired by focusing the beam on the ROI 910, the enlarged version 920 may suffer little loss of image quality despite its enlargement beyond a normal image size.

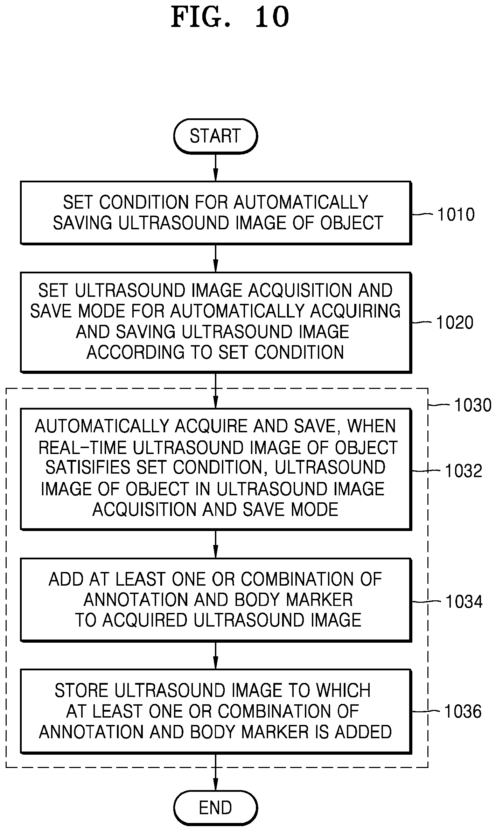

FIG. 10 is a flowchart of a method, performed by the ultrasound imaging apparatus 300, of automatically saving an ultrasound image by adding an annotation or body marker thereto, according to some embodiments.

Referring to FIG. 10, the ultrasound imaging apparatus 300 may set a condition for automatically saving an ultrasound image of an object (operation 1010). According to some embodiments, operation 1010 may correspond to operation 410 described with reference to FIG. 4.

The ultrasound imaging apparatus 300 may set an ultrasound image acquisition and save mode for automatically acquiring and saving the ultrasound image of the object according to the set condition (operation 1020). According to some embodiments, operation 1020 may correspond to operation 420 described with reference to FIG. 4.

When a real-time ultrasound image of the object satisfies the set condition, the ultrasound imaging apparatus 300 may automatically acquire and save the ultrasound image of the object based on the ultrasound image acquisition and save mode (operation 1030). Operation 1030 may include operations 1032, 1034, and 1036.

According to some embodiments, operation 1030 may correspond to operation 430 described with reference to FIG. 4. For convenience, descriptions corresponding to operation 430 of FIG. 4 will be omitted hereinafter, and only a difference from operation 430 is described.

In detail, when a real-time ultrasound image of the object satisfies the set condition, the ultrasound imaging apparatus 300 may automatically acquire and save an ultrasound image of the object based on the ultrasound image acquisition and save mode (operation 1032).

The ultrasound imaging apparatus 300 may add at least one or a combination of an annotation and a body marker to the acquired ultrasound image (operation 1034).

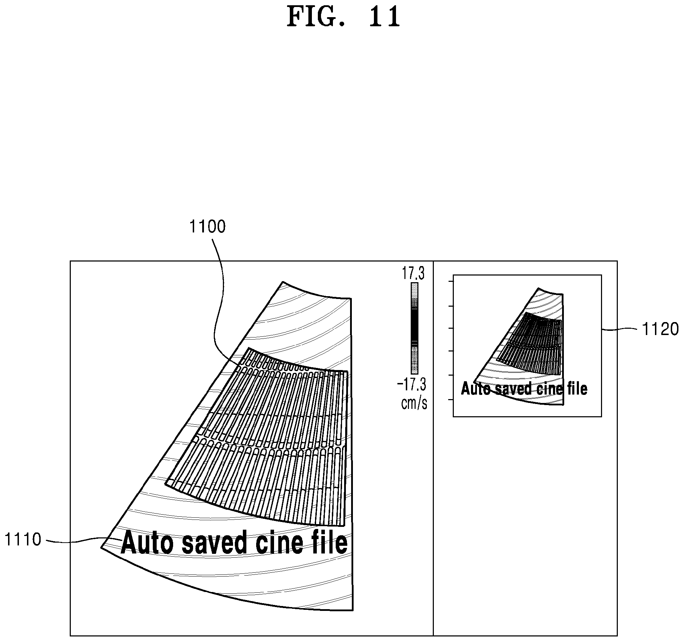

According to some embodiments, the ultrasound imaging apparatus 300 may add an annotation to the acquired ultrasound image. The annotation may be related to the acquired ultrasound image or may be input according to a user input. Referring to FIG. 11, the ultrasound imaging apparatus 300 may obtain an ultrasound image 1100 of an object and add to the ultrasound image 1100 at least one or a combination of information about whether the obtained ultrasound image 1100 is an automatically saved image and information about an auto save condition for the ultrasound image 1100. For example, the ultrasound imaging apparatus 300 may add to the obtained ultrasound image 1100 an annotation 1110 such as 'Auto saved cine file' indicating that the saved image is an automatically saved cine image. Furthermore, when the obtained ultrasound image 1100 is an ultrasound image acquired according to an auto save condition based on a velocity of blood flow, the ultrasound imaging apparatus 300 may add to the obtained ultrasound image 1100 an annotation indicating that the saved image is an image in which a velocity of blood flow is greater than a specific value.

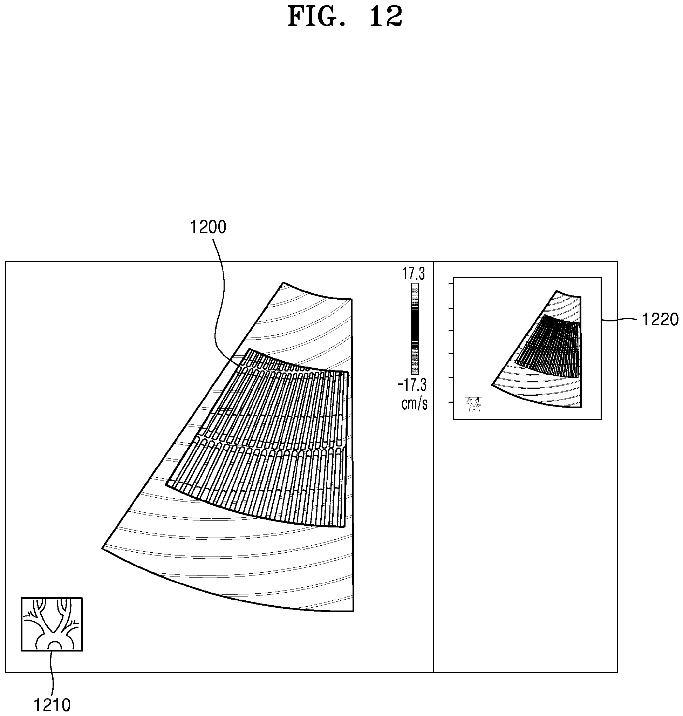

According to some embodiments, the ultrasound imaging apparatus 300 may add a body marker to an obtained ultrasound image. A body marker is an image indicator displayed in a medical image, and may be shown as a picture or icon in the medical image to indicate which body part (e.g., the heart, liver, stomach, kidneys, uterus, etc.) of the object is measured in the medical image or the top, bottom, left and right sides of the measured body part of the object. Referring to FIG. 12, the ultrasound imaging apparatus 300 may acquire an ultrasound image 1200 of an object and add a body marker 1210 indicating that the ultrasound image is associated with the heart to the acquired ultrasound image 1200. The ultrasound imaging apparatus 300 may detect a part of the object by recognizing segmentation of the object in the ultrasound image 1200 and add the body marker 1210 to the ultrasound image 1200. According to another embodiment, the ultrasound imaging apparatus 300 may add a body marker to an ultrasound image based on a user input.

According to some embodiments, the ultrasound imaging apparatus 300 may add both an annotation and a body marker to the acquired ultrasound image.

According to some embodiments, the ultrasound imaging apparatus 300 may set whether to add a body marker or annotation to an ultrasound image to be automatically saved. The setting with regard to whether to add a body marker or annotation to the ultrasound image to be automatically saved may be performed by the ultrasound imaging apparatus 300 before operation 1036. The ultrasound imaging apparatus 300 may add, based on the setting, a body marker or annotation to the ultrasound image to be automatically saved.

The ultrasound imaging apparatus 300 may store the ultrasound image to which at least one or a combination of the annotation and the body marker is added (operation 1036).

According to some embodiments, the ultrasound imaging apparatus 300 may save an ultrasound image having an annotation added thereto. Referring to FIG. 11, the ultrasound imaging apparatus 300 may save an ultrasound image 1120 having an annotation added thereto.

According to some embodiments, the ultrasound imaging apparatus 300 may save an ultrasound image to which a body marker is added. Referring to FIG. 12, the ultrasound imaging apparatus 300 may save an ultrasound image 1220 to which a body marker is added.

According to some embodiments, the ultrasound imaging apparatus 300 may save an ultrasound image to which both an annotation and a body marker are added.

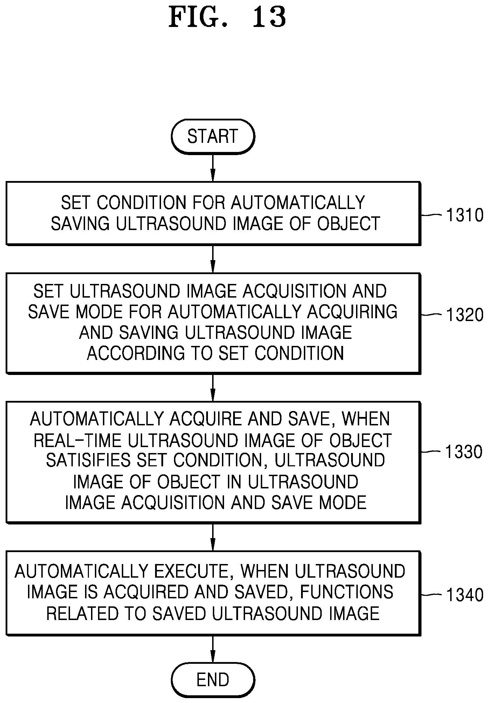

FIG. 13 is a flowchart of a method, performed by an ultrasound imaging apparatus, of automatically saving an ultrasound image and automatically executing a function related to the saved ultrasound image, according to some embodiments.

Referring to FIG. 13, the ultrasound imaging apparatus 300 may set a condition for automatically saving an ultrasound image of an object (operation 1310). According to some embodiments, operation 1310 may correspond to operation 410 described with reference to FIG. 4.

The ultrasound imaging apparatus 300 may set an ultrasound image acquisition and save mode for automatically acquiring and saving the ultrasound image of the object according to the set condition (operation 1320). According to some embodiments, operation 1320 may correspond to operation 420 described with reference to FIG. 4.

When a real-time ultrasound image of the object satisfies the set condition, the ultrasound imaging apparatus 300 may automatically acquire and save the ultrasound image of the object based on the ultrasound image acquisition and save mode (operation 1330). According to some embodiments, operation 1330 may correspond to operation 430 described with reference to FIG. 4.

As the ultrasound image is acquired and saved, the ultrasound imaging apparatus 300 may automatically execute functions related to the saved ultrasound image (operation 1340).

According to some embodiments, the functions related to the saved ultrasound image may include a diagnostic function with respect to an object in the saved ultrasound image. For example, when an ultrasound image of the heart is automatically saved, the ultrasound imaging apparatus 300 may automatically perform a function for diagnosing the heart of the object in the saved ultrasound image of the heart.

According to some embodiments, the functions related to the saved ultrasound image may include a measurement function with respect to the saved ultrasound image. For example, when an ultrasound image of the heart is automatically saved, the ultrasound imaging apparatus 300 may automatically perform a function for measuring a region of blood flow in the saved ultrasound image of the heart.

According to some embodiments, the functions related to the saved ultrasound image may include an additional function determined according to an audio save condition for the ultrasound image. For example, when the ultrasound image is saved as it satisfies an auto save condition set based on a velocity of blood flow, the functions related to the saved ultrasound image may include a function for automatically measuring a velocity of blood flow in an ROI in the ultrasound image, a function for displaying the automatically measured velocity of blood flow, a function for determining an image exhibiting a highest blood flow velocity in an ROI, etc.

According to some embodiments, when the saved ultrasound image is a cine image of the heart, the functions related to the saved ultrasound image may include a function for detecting a still image captured of a largest reverse blood flow region in the saved cine image and displaying the detected still image in a cine frame.

According to some embodiments, the functions related to the saved ultrasound image may include a function for changing a preset on the ultrasound imaging apparatus 300. For example, when the ultrasound imaging apparatus 300 automatically saves an ultrasound image, a function for changing a preset (e.g., displaying an interface for changing a preset) may be automatically executed.

According to some embodiments, when an ultrasound image is acquired and saved, the ultrasound imaging apparatus 300 may set functions to be automatically executed. A setting with respect to the functions to be automatically executed may be performed by the ultrasound imaging apparatus 300 before operation 1340. When the ultrasound image is automatically acquired and saved, the ultrasound imaging apparatus 300 may execute functions related to the automatically saved ultrasound image based on the setting.

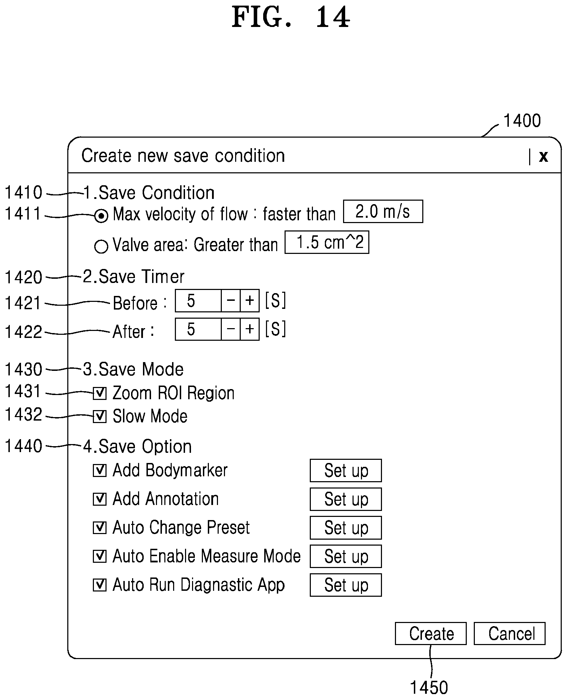

FIG. 14 illustrates an example in which the ultrasound imaging apparatus 300 sets a condition for automatically saving an ultrasound image and an ultrasound image acquisition and save mode for automatically acquiring and saving the ultrasound image, according to some embodiments.

Referring to FIG. 14, the ultrasound imaging apparatus 300 may display a UI 1400 designed to set a condition for automatically saving an ultrasound image and an ultrasound image acquisition and save mode for automatically acquiring and saving the ultrasound image. The ultrasound imaging apparatus 300 may receive a user input via the displayed UI 1400. The ultrasound imaging apparatus 300 may set, based on the user input received via the UI 1400, the condition for automatically saving an ultrasound image and the ultrasound image acquisition and save mode for automatically acquiring and saving the ultrasound image.

For example, the ultrasound imaging apparatus 300 may receive a user input with regard to an item 1410 for setting a condition for automatically saving an ultrasound image and set the auto save condition based on the received user input. Referring to FIG. 14, a case 1411 wherein a velocity of blood flow included in an ROI of an ultrasound image is greater than 2 m/s is set as the auto save condition.

Furthermore, the ultrasound imaging apparatus 300 receive user inputs with regard to items 1420 and 1430 for setting an ultrasound image acquisition and save mode and set the ultrasound image acquisition and save mode based on the received user inputs. Referring to FIG. 14, time periods 1421 and 1422 during which an ultrasound image of an object to be automatically saved is obtained are respectively set to -5s and +5s from a time point when a real-time ultrasound image satisfying the save condition is acquired. As seen on FIG. 14, the ultrasound image acquisition and save mode is set to acquire and save an enlarged version of an image of a portion corresponding to an ROI in the obtained ultrasound image (1431) and acquire and save the ultrasound image of the object at a high frame rate (1432).

Furthermore, the ultrasound imaging apparatus 300 may receive a user input for other setting items 1440 on the UI 1400 and set whether to add a body marker, an annotation, etc., to an ultrasound image to be saved based on the received user input. Furthermore, when the ultrasound image is automatically saved based on the received user input, the ultrasound imaging apparatus 300 may set functions to be automatically executed in association with the saved ultrasound image.

The ultrasound imaging apparatus 300 may store a setting with respect to a condition for automatically saving an ultrasound image, a setting with respect to an ultrasound image acquisition and save mode, and other settings related to automatic saving of the ultrasound image. The ultrasound imaging apparatus 300 may set the condition for automatically saving an ultrasound image, an ultrasound image acquisition and save mode, etc., and save setting results as a single setting for automatically saving an ultrasound image.

For example, the ultrasound imaging apparatus 300 may set a condition for automatically saving an ultrasound image, an ultrasound image acquisition and save mode, etc., via the UI 1400 and save setting results as a single setting for automatically saving an ultrasound image (1450).

Settings for automatically saving an ultrasound image may be stored as a list 1500. When one setting is selected from the list 1500 of settings for automatically saving an ultrasound image, the ultrasound imaging apparatus 300 may automatically save the ultrasound image according to the selected setting.

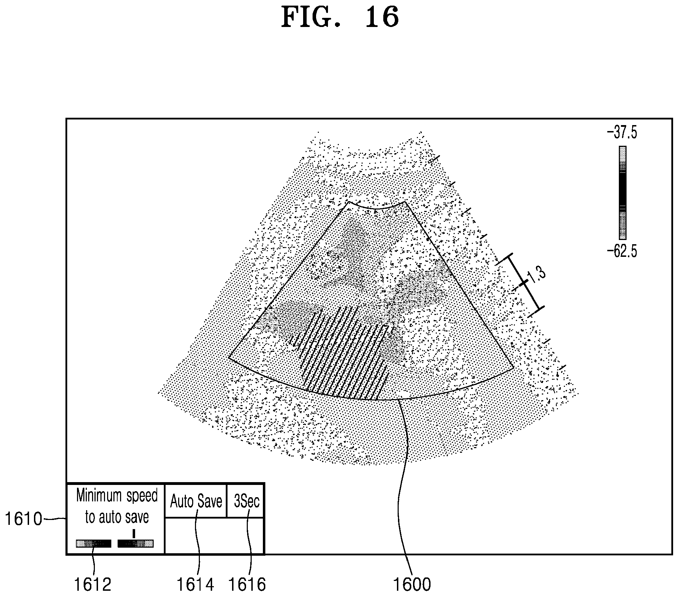

FIG. 16 illustrates an example in which the ultrasound imaging apparatus 300 changes in real-time a condition for automatically saving an ultrasound image and an ultrasound image acquisition and save mode for automatically acquiring and saving the ultrasound image, according to some embodiments.

Referring to FIG. 16, the ultrasound imaging apparatus 300 may display a real-time ultrasound image 1600 of an object while simultaneously displaying a UI 1610 for changing in real-time a condition for automatically saving an ultrasound image and an ultrasound image acquisition and save mode for automatically acquiring and saving the ultrasound image.

For example, when a condition for automatically saving an ultrasound image is set based on a velocity of blood flow, the ultrasound imaging apparatus 300 may change the velocity of blood flow in real-time. The UI 1610 may include a color bar 1620 corresponding to a velocity of blood flow in the real-time ultrasound image 1600 that is a color Doppler ultrasound image. The ultrasound imaging apparatus 300 may change or set in real-time the velocity of blood flow that is used as a reference for automatically saving an ultrasound image, based on a user input with respect to the color bar 1620.

Furthermore, the UI 1610 may further include an interface 1614 for changing or setting whether to automatically save an ultrasound image, an interface 1616 for setting an ultrasound image acquisition time period, etc. The ultrasound imaging apparatus 300 may respectively change or set in real-time whether to automatically save an ultrasound image and a time period during which an ultrasound image to be automatically saved is acquired via the interfaces 1614 and 1616.

The UI 1610 shown in FIG. 16 is merely an example, and embodiments are not limited thereto. For example, even when a condition for automatically saving an ultrasound image is set based on an area of a valve orifice or a size of a regurgitant jet area as well as a velocity of blood flow, the ultrasound imaging apparatus 300 may change settings in real-time via a UI corresponding to the set condition. Furthermore, the ultrasound imaging apparatus 300 may change in real-time a setting with respect to a case where an ultrasound image of an object is saved at a high frame rate or an enlarged version of an ultrasound image is saved via a corresponding UI.

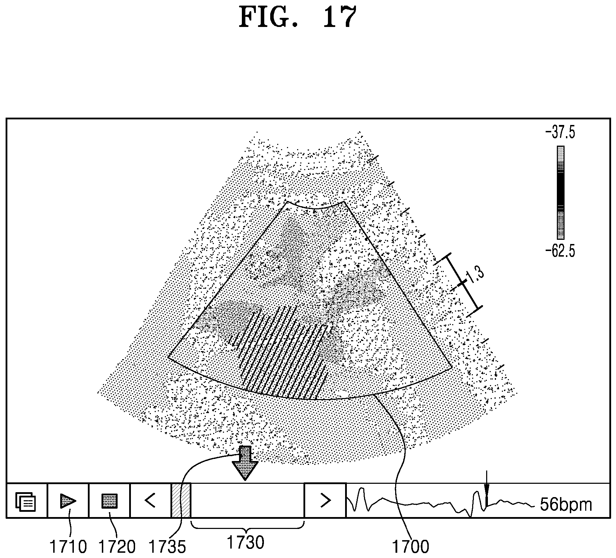

FIG. 17 illustrates an example in which the ultrasound imaging apparatus 300 plays back an automatically saved cine image, according to some embodiments.

Referring to FIG. 17, when an automatically saved ultrasound image is a cine image 1700, the ultrasound imaging apparatus 300 may play back the cine image. The ultrasound imaging apparatus 300 may display a UI for playing back the cine image 1700, together with the cine image 1700. For example, the ultrasound imaging apparatus 300 may display a replay button 1710, a stop button 1720, etc., as a UI for playing back the cine image 1700. Furthermore, the ultrasound imaging apparatus 300 may display a UI 1730 representing an interval at which the cine image 1700 is played back.

According to an embodiment, the ultrasound imaging apparatus 300 may display a frame in which a size of a regurgitant jet area is the largest in the cine image 1700, a frame in which a velocity of blood flow is the highest, etc., on the UI 1730 representing the playback interval of the cine image 1700 (1735).

According to an embodiment, the ultrasound imaging apparatus 300 may play back the cine image 1700 at a specific frame rate. For example, the ultrasound imaging apparatus 300 may play back the cine image 1700 at a frame rate at which the cine image is 1700 is acquired or at a frame rate less or greater than the acquisition frame rate. Furthermore, the ultrasound imaging apparatus 300 may play back the cine image 1700 in a slow mode.

According to the embodiment, the frame in which a size of a regurgitant jet area is largest and the frame in which a velocity of blood flow is highest may be displayed on the UI 1730 to allow a user to more easily view an image related to a lesion to be diagnosed in the cine image 1700. Furthermore, according to the embodiment, by playing back the cine image 1700 of an object in a slow mode, it is possible to more precisely examine an image of the object to be diagnosed.

The embodiments of the disclosure may be implemented as a software program including instructions stored in computer-readable storage media.

A computer may refer to a device capable of retrieving instructions stored in the computer-readable storage media and performing operations according to embodiments in response to the retrieved instructions, and may include ultrasound imaging apparatuses 100, 100a, 100b, 100c, and 300 according to the embodiments.

The computer-readable storage media may be provided in the form of non-transitory storage media. In this case, the term 'non-transitory' only means that the storage media do not include signals and are tangible, and the term does not distinguish between data that is semi-permanently stored and data that is temporarily stored in the storage media.

In addition, apparatuses and methods of automatically saving an ultrasound image according to embodiments of the disclosure may be included in a computer program product when provided. The computer program product may be traded, as a commodity, between a seller and a buyer.

The computer program product may include a software program and a computer-readable storage medium having stored thereon the software program. For example, the computer program product may include a product (e.g. a downloadable application) in the form of a software program electronically distributed by a manufacturer of an ultrasound diagnosis apparatus or through an electronic market (e.g., Google Play Store ^{™}, and App Store ^{™}). For such electronic distribution, at least a part of the software program may be stored on the storage medium or may be temporarily generated. In this case, the storage medium may be a storage medium of a server of the manufacturer, a server of the electronic market, or a relay server for temporarily storing the software program.

In a system consisting of a server and a device (e.g., an ultrasound imaging apparatus), the computer program product may include a storage medium of the server or a storage medium of the device. Alternatively, in a case where a third device (e.g., a smartphone) is connected to the server or device through a communication network, the computer program product may include a storage medium of the third device. Alternatively, the computer program product may include a software program that is transmitted from the server to the device or the third device or that is transmitted from the third device to the device.

In this case, one of the server, the device, and the third device may execute the computer program product to perform the methods according to embodiments of the disclosure. Alternatively, two or more of the server, the device, and the third device may execute the computer program product to perform the methods according to the embodiments in a distributed manner.

For example, the server (e.g., a cloud server, an artificial intelligence (AI) server, or the like) may run the computer program product stored therein to control the device communicating with the server to perform the methods according to the embodiments.

As another example, the third device may execute the computer program product to control the device communicating with the third device to perform the methods according to the embodiments. In detail, the third device may remotely control an ultrasound system to transmit ultrasound signals to an object and generate an ultrasound image of an inner part of the object based on echo signals corresponding to the transmitted ultrasound signals.

In a case where the third device executes the computer program product, the third device may download the computer program product from the server and execute the downloaded computer program product. Alternatively, the third device may execute the computer program product that is pre-loaded therein to perform the methods according to the embodiments.

While embodiments of the disclosure have been particularly shown and described with reference to the accompanying drawings, it will be understood by those of ordinary skill in the art that various changes in form and details may be made as far as they fall under the scope of the appended claims. The disclosed embodiments should be considered in a descriptive sense only and not for purposes of limitation.

## Claims

1. An ultrasound imaging apparatus capable of automatically saving an ultrasound image of an object, the ultrasound imaging apparatus comprising:
a probe (310) configured to acquire the ultrasound image of the object;
a memory (320) storing the ultrasound image of the object; and
at least one processor (340) configured to set a condition for automatically saving the ultrasound image of the object, set an ultrasound image acquisition and save mode for automatically acquiring and saving the ultrasound image of the object according to the condition, and automatically acquire and save, when a real-time ultrasound image of the object satisfies the condition, the ultrasound image of the object based on the ultrasound image acquisition and save mode,
wherein the condition comprises a condition where a velocity of blood flow in a region of interest in the real-time ultrasound image of the object is greater than a particular value, and
wherein the ultrasound image acquisition and save mode comprises automatically acquiring and saving an enlarged image of the region of interest, and wherein the at least one processor is further configured to automatically acquire the enlarged image of the region of interest by focusing a beam on the region of interest and automatically save the acquired enlarged image, when the real-time ultrasound image of the object satisfies the condition.

2. The ultrasound imaging apparatus of claim 1, wherein the condition further comprises a condition where an area of a valve orifice in the region of interest in the real-time ultrasound image of the object is greater than a particular value.

3. The ultrasound imaging apparatus of claim 1, wherein the condition further comprises a condition where a size of a regurgitant jet area in the region of interest in the real-time ultrasound image of the object is greater than a particular value.

4. The ultrasound imaging apparatus of claim 1, wherein the at least one processor is further configured to set the ultrasound image acquisition and save mode to acquire the ultrasound image of the object at a predetermined frame rate or higher and save the acquired ultrasound image of the object.

5. The ultrasound imaging apparatus of claim 1, wherein the at least one processor is further configured to set the ultrasound image acquisition and save mode by setting a time period during which the ultrasound image of the object to be automatically saved is acquired , and
acquire, when the real-time ultrasound image of the object satisfies the condition, the ultrasound image of the object during the set time period based on the ultrasound image acquisition and save mode and save the ultrasound image acquired during the set time period as a cine image.

6. A method of operating an ultrasound imaging apparatus configured to automatically save an ultrasound image of an object, the method comprising:
setting a condition for automatically saving the ultrasound image of the object (410);
setting an ultrasound image acquisition and save mode for automatically acquiring and saving the ultrasound image of the object according to the condition (420); and
automatically acquiring and saving, when a real-time ultrasound image of the object satisfies the condition, the ultrasound image of the object based on the ultrasound image acquisition and save mode (430),
wherein the condition comprises a condition where a velocity of blood flow in a region of interest in the real-time ultrasound image of the object is greater than a particular value, and
wherein the ultrasound image acquisition and save mode comprises automatically acquiring and saving an enlarged image of the region of interest, and
wherein the automatic acquiring and saving of the ultrasound image of the object comprises automatically acquiring the enlarged image of the region of interest by focusing a beam on the region of interest and automatically saving the acquired enlarged image, when the real-time ultrasound image of the object satisfies the condition.

7. The method of claim 6, wherein the condition further comprises a condition where an area of a valve orifice in a region of interest in the real-time ultrasound image of the object is greater than a particular value.

8. The method of claim 6, wherein the condition further comprises a condition where a size of a regurgitant jet area in a region of interest in the real-time ultrasound image of the object is greater than a particular value.

9. The method of claim 6, wherein the setting of the ultrasound image acquisition and save mode comprises setting the ultrasound image acquisition and save mode to acquire the ultrasound image of the object at a predetermined frame rate or higher and save the acquired ultrasound image of the object.

10. The method of claim 6, wherein the setting of the ultrasound image acquisition and save mode comprises setting a time period during which the ultrasound image of the object to be automatically saved is acquired, and
wherein the automatic acquiring and saving of the ultrasound image of the object comprises:
acquiring the ultrasound image of the object during the set time period in the ultrasound image acquisition and save mode; and
saving the ultrasound image acquired during the set time period as a cine image.

11. A computer program product comprising a recording medium having stored therein program instructions that, when executed by a processor of an ultrasound imaging apparatus, cause the processor to perform a method of automatically saving an ultrasound image of an object, the method comprising:
setting a condition for automatically saving the ultrasound image of the object;
setting an ultrasound image acquisition and save mode for automatically acquiring and saving the ultrasound image of the object according to the condition; and
automatically acquiring and saving, when a real-time ultrasound image of the object satisfies the condition, the ultrasound image of the object based on the ultrasound image acquisition and save mode,
wherein the condition comprises a condition where a velocity of blood flow in a region of interest in the real-time ultrasound image of the object is greater than a particular value,
wherein the ultrasound image acquisition and save mode comprises automatically acquiring and saving an enlarged image of the region of interest, and
wherein the automatic acquiring and saving of the ultrasound image of the object comprises automatically acquiring the enlarged image of the region of interest by focusing a beam on the region of interest and automatically saving the acquired enlarged image, when the real-time ultrasound image of the object satisfies the condition.

## Patentansprüche

1. Ultraschallbildgebungsvorrichtung, die automatisch ein Ultraschallbild eines Objekts speichern kann, wobei die Ultraschallbildgebungsvorrichtung Folgendes umfasst:
eine Sonde (310), die dazu konfiguriert ist, das Ultraschallbild des Objekts zu erfassen;
einen Speicher (320), der das Ultraschallbild des Objekts speichert; und
mindestens einen Prozessor (340), der dazu konfiguriert ist, eine Bedingung zum automatischen Speichern des Ultraschallbilds des Objekts einzustellen, einen Ultraschallbilderfassungs- und Speichermodus zum automatischen Erfassen und Speichern des Ultraschallbilds des Objekts gemäß der Bedingung einzustellen, und wenn ein Echtzeit-Ultraschallbild des Objekts die Bedingung erfüllt, das Ultraschallbild des Objekts basierend auf dem Ultraschallbilderfassungs- und Speichermodus automatisch zu erfassen und zu speichern,
wobei die Bedingung eine Bedingung umfasst, bei der die Blutflussgeschwindigkeit in einem Bereich von Interesse in dem Echtzeit-Ultraschallbild des Objekts größer als ein bestimmter Wert ist, und
wobei der Ultraschallbilderfassungs- und Speichermodus das automatische Erfassen und Speichern eines vergrößerten Bilds des Bereichs von Interesse umfasst, und
wobei der mindestens eine Prozessor weiterhin dazu konfiguriert ist, das vergrößerte Bild des Bereichs von Interesse automatisch zu erfassen, indem ein Strahl auf einen Bereich von Interesse fokussiert wird, und das erfasste vergrößerte Bild automatisch zu speichern, wenn das Echtzeit-Ultraschallbild des Objekts die Bedingung erfüllt.

2. Ultraschallbildgebungsvorrichtung nach Anspruch 1, wobei die Bedingung weiterhin eine Bedingung umfasst, bei der die Fläche einer Klappenöffnung in dem Bereich von Interesse in dem Echtzeit-Ultraschallbild des Objekts größer als ein bestimmter Wert ist.

3. Ultraschallbildgebungsvorrichtung nach Anspruch 1, wobei die Bedingung weiterhin eine Bedingung umfasst, bei der die Größe einer Insuffizienzstrahlfläche in dem Bereich von Interesse in dem Echtzeit-Ultraschallbild des Objekts größer als ein bestimmter Wert ist.

4. Ultraschallbildgebungsvorrichtung nach Anspruch 1, wobei der mindestens eine Prozessor weiterhin dazu konfiguriert ist, der Ultraschallbilderfassungs- und Speichermodus so einzustellen, dass das Ultraschallbild des Objekts mit einer vorbestimmten Bildfrequenz oder höher erfasst wird und das erfasste Ultraschallbild des Objekts gespeichert wird.

5. Ultraschallbildgebungsvorrichtung nach Anspruch 1, wobei der mindestens eine Prozessor weiterhin dazu konfiguriert ist, den Ultraschallbilderfassungs- und Speichermodus einzustellen, indem er einen Zeitraum einstellt, während dessen das automatisch zu speichernde Ultraschallbild des Objekts erfasst wird, und
wenn das Echtzeit-Ultraschallbild des Objekts die Bedingung erfüllt, das Ultraschallbild des Objekts während des eingestellten Zeitraums basierend auf dem Ultraschallbilderfassungs- und Speichermodus zu erfassen und das während des eingestellten Zeitraums erfasste Ultraschallbild als ein Cine-Bild zu speichern.

6. Verfahren zum Betreiben einer Ultraschallbildgebungsvorrichtung, die dazu konfiguriert ist, automatisch ein Ultraschallbild eines Objekts zu speichern, wobei das Verfahren Folgendes umfasst:
Einstellen einer Bedingung zum automatischen Speichern des Ultraschallbilds des Objekts (410);
Einstellen eines Ultraschallbilderfassungs- und Speichermodus zum automatischen Erfassen und Speichern des Ultraschallbilds des Objekts gemäß der Bedingung (420); und
wenn ein Echtzeit-Ultraschallbild des Objekts die Bedingung erfüllt, automatisches Erfassen und Speichern des Ultraschallbilds des Objekts basierend auf dem Ultraschallbilderfassungs- und Speichermodus (430),
wobei die Bedingung eine Bedingung umfasst, bei der die Blutflussgeschwindigkeit in einem Bereich von Interesse in dem Echtzeit-Ultraschallbild des Objekts größer als ein bestimmter Wert ist,
wobei der Ultraschallbilderfassungs- und Speichermodus das automatische Erfassen und Speichern eines vergrößerten Bilds des Bereichs von Interesse umfasst, und
wobei das automatische Erfassen und Speichern des Ultraschallbilds des Objekts Folgendes umfasst: automatisches Erfassen des vergrößerten Bilds des Bereichs von Interesse, indem ein Strahl auf dem Bereich von Interesse fokussiert wird, und automatisches Speichern des erfassten vergrößerten Bilds, wenn das Echtzeit-Ultraschallbild des Objekts die Bedingung erfüllt.

7. Verfahren nach Anspruch 6, wobei die Bedingung weiterhin eine Bedingung umfasst, bei der die Fläche einer Klappenöffnung in einem Bereich von Interesse in dem Echtzeit-Ultraschallbild des Objekts größer als ein bestimmter Wert ist.

8. Verfahren nach Anspruch 6, wobei die Bedingung weiterhin eine Bedingung umfasst, bei der die Größe einer Insuffizienzstrahlfläche in einem Bereich von Interesse in dem Echtzeit-Ultraschallbild des Objekts größer als ein bestimmter Wert ist.

9. Verfahren nach Anspruch 6, wobei das Einstellen des Ultraschallbilderfassungs- und Speichermodus Folgendes umfasst: das Einstellen des Ultraschallbilderfassungs- und Speichermodus, so dass das Ultraschallbild des Objekts mit einer vorbestimmten Bildfrequenz oder höher erfasst wird und das erfasste Ultraschallbild des Objekts gespeichert wird.

10. Verfahren nach Anspruch 6, wobei das Einstellen des Ultraschallbilderfassungs- und Speichermodus Folgendes umfasst: Einstellen eines Zeitraums während dessen das automatisch zu speichernde Ultraschallbild des Objekts erfasst wird, und
wobei das automatische Erfassen und Speichern des Ultraschallbilds des Objekts Folgendes umfasst:
Erfassen des Ultraschallbilds des Objekts in dem Ultraschallbilderfassungs- und Speichermodus während des eingestellten Zeitraums; und
Speichern des während des eingestellten Zeitraums erfassten Ultraschallbilds als ein Cine-Bild.

11. Computerprogrammprodukt, umfassend ein Aufzeichnungsmedium mit darauf gespeicherten Programmanweisungen, die, wenn sie von einem Prozessor einer Ultraschallbildgebungsvorrichtung ausgeführt werden, bewirken, dass der Prozessor ein Verfahren zum automatischen Speichern eines Ultraschallbilds eines Objekts durchführt, wobei das Verfahren Folgendes umfasst:
Einstellen einer Bedingung zum automatischen Speichern des Ultraschallbilds des Objekts;
Einstellen eines Ultraschallbilderfassungs- und Speichermodus zum automatischen Erfassen und Speichern des Ultraschallbilds des Objekts gemäß der Bedingung; und
wenn ein Echtzeit-Ultraschallbild des Objekts die Bedingung erfüllt, automatisches Erfassen und Speichern des Ultraschallbilds des Objekts basierend auf dem Ultraschallbilderfassungs- und Speichermodus,
wobei die Bedingung eine Bedingung umfasst, bei der die Blutflussgeschwindigkeit in einem Bereich von Interesse in dem Echtzeit-Ultraschallbild des Objekts größer als ein bestimmter Wert ist,
wobei der Ultraschallbilderfassungs- und Speichermodus das automatische Erfassen und Speichern eines vergrößerten Bilds des Bereichs von Interesse umfasst, und
wobei das automatische Erfassen und Speichern des Ultraschallbilds des Objekts Folgendes umfasst: automatisches Erfassen des vergrößerten Bilds des Bereichs von Interesse, indem ein Strahl auf dem Bereich von Interesse fokussiert wird, und automatisches Speichern des erfassten vergrößerten Bilds, wenn das Echtzeit-Ultraschallbild des Objekts die Bedingung erfüllt.

## Revendications

1. Appareil échographique capable de sauvegarder automatiquement une image échographique d'un objet, l'appareil échographique comprenant :
une sonde (310) conçue pour acquérir l'image échographique de l'objet,
une mémoire (320) dans laquelle est stockée l'image échographique de l'objet, et
au moins un processeur (340) conçu pour définir une condition de sauvegarde automatique de l'image échographique de l'objet, définir un mode d'acquisition et de sauvegarde d'image échographique permettant d'acquérir et de sauvegarder automatiquement l'image échographique de l'objet conformément à la condition, et, lorsqu'une image échographique en temps réel de l'objet satisfait la condition, acquérir et sauvegarder automatiquement l'image échographique de l'objet compte tenu du mode d'acquisition et de sauvegarde d'image échographique ;
ladite condition comprenant une condition dans laquelle la vitesse du flux sanguin dans une région d'intérêt de l'image échographique en temps réel de l'objet est supérieure à une valeur particulière, et
ledit mode d'acquisition et de sauvegarde d'image échographique comprenant l'acquisition et la sauvegarde automatiques d'une image agrandie de la région d'intérêt, et
ledit au moins un processeur étant conçu en outre pour acquérir automatiquement l'image agrandie de la région d'intérêt par concentration d'un faisceau sur la région d'intérêt et pour sauvegarder automatiquement l'image agrandie, lorsque l'image échographique en temps réel de l'objet satisfait la condition.

2. Appareil échographique selon la revendication 1, dans lequel la condition comprend en outre une condition dans laquelle l'aire d'une ouverture de valvule dans la région d'intérêt de l'image échographique en temps réel de l'objet est supérieure à une valeur particulière.

3. Appareil échographique selon la revendication 1, dans lequel la condition comprend en outre une condition dans laquelle la taille de l'aire d'un jet de régurgitation dans la région d'intérêt de l'image échographique en temps réel de l'objet est supérieure à une valeur particulière.

4. Appareil échographique selon la revendication 1, dans lequel l'au moins un processeur est conçu en outre pour définir le mode d'acquisition et de sauvegarde d'image échographique pour acquérir l'image échographique de l'objet selon une fréquence d'image prédéterminée ou supérieure et pour sauvegarder l'image échographique de l'objet acquise.

5. Appareil échographique selon la revendication 1, dans lequel l'au moins un processeur est conçu en outre pour définir le mode d'acquisition et de sauvegarde d'image échographique par définition d'une période pendant laquelle l'image échographique de l'objet destinée à être sauvegardée automatiquement est acquise, et
lorsque l'image échographique en temps réel de l'objet satisfait la condition, pour acquérir l'image échographique de l'objet pendant la période définie, compte tenu du mode d'acquisition et de sauvegarde d'image échographique, et sauvegarder l'image échographique acquise pendant la période définie, sous la forme d'une image ciné.

6. Procédé d'exploitation d'un appareil échographique conçu pour sauvegarder automatiquement une image échographique d'un objet, le procédé comprenant :
la définition d'une condition de sauvegarde automatique de l'image échographique de l'objet (410), la définition d'un mode d'acquisition et de sauvegarde d'image échographique permettant d'acquérir et de sauvegarder automatiquement l'image échographique de l'objet conformément à la condition (420), et
lorsqu'une image échographique en temps réel de l'objet satisfait la condition, l'acquisition et la sauvegarde automatiques de l'image échographique de l'objet compte tenu du mode d'acquisition et de sauvegarde d'image échographique (430) ;
ladite condition comprenant une condition dans laquelle la vitesse du flux sanguin dans une région d'intérêt de l'image échographique en temps réel de l'objet est supérieure à une valeur particulière, ledit mode d'acquisition et de sauvegarde d'image échographique comprenant l'acquisition et la sauvegarde automatiques d'une image agrandie de la région d'intérêt, et
lesdites acquisition et sauvegarde automatiques de l'image échographique de l'objet comprenant l'acquisition automatique de l'image agrandie de la région d'intérêt par concentration d'un faisceau sur la région d'intérêt et la sauvegarde automatique de l'image agrandie acquise, lorsque l'image échographique en temps réel de l'objet satisfait la condition.

7. Procédé selon la revendication 6, dans lequel la condition comprend en outre une condition dans laquelle l'aire d'une ouverture de valvule dans une région d'intérêt de l'image échographique en temps réel de l'objet est supérieure à une valeur particulière.

8. Procédé selon la revendication 6, dans lequel la condition comprend en outre une condition dans laquelle la taille de l'aire d'un jet de régurgitation dans une région d'intérêt de l'image échographique en temps réel de l'objet est supérieure à une valeur particulière.

9. Procédé selon la revendication 6, dans lequel la définition du mode d'acquisition et de sauvegarde d'image échographique comprend la définition du mode d'acquisition et de sauvegarde d'image échographique de façon à acquérir l'image échographique de l'objet selon une fréquence d'image prédéterminée ou supérieure et à sauvegarder l'image échographique de l'objet acquise.

10. Procédé selon la revendication 6, dans lequel la définition du mode d'acquisition et de sauvegarde d'image échographique comprend la définition d'une période pendant laquelle l'image échographique de l'objet destinée à être sauvegardée automatiquement est acquise, et
dans lequel l'acquisition et la sauvegarde automatiques de l'image échographique de l'objet comprend :
l'acquisition de l'image échographique de l'objet pendant la période définie, sous le mode d'acquisition et de sauvegarde d'image échographique, et
la sauvegarde de l'image échographique acquise pendant la période définie, sous la forme d'une image ciné.

11. Produit-programme d'ordinateur comprenant un support d'enregistrement sur lequel sont enregistrées des instructions de programme qui, lorsqu'elles sont exécutées par un processeur d'un appareil échographique, amènent le processeur à réaliser un procédé permettant de sauvegarder automatiquement une image échographique d'un objet, le procédé comprenant :
la définition d'une condition de sauvegarde automatique de l'image échographique de l'objet ;
la définition d'un mode d'acquisition et de sauvegarde d'image échographique permettant d'acquérir et de sauvegarder automatiquement l'image échographique de l'objet conformément à la condition ; et
lorsqu'une image échographique en temps réel de l'objet satisfait la condition, l'acquisition et la sauvegarde automatiques de l'image échographique de l'objet compte tenu du mode d'acquisition et de sauvegarde d'image échographique,
ladite condition comprenant une condition dans laquelle la vitesse du flux sanguin dans une région d'intérêt de l'image échographique en temps réel de l'objet est supérieure à une valeur particulière, ledit mode d'acquisition et de sauvegarde d'image échographique comprenant l'acquisition et la sauvegarde automatiques d'une image agrandie de la région d'intérêt, et
l'acquisition et la sauvegarde automatiques de l'image échographique de l'objet comprend l'acquisition automatique de l'image agrandie de la région d'intérêt par concentration d'un faisceau sur la région d'intérêt et la sauvegarde automatique de l'image agrandie acquise, lorsque l'image échographique en temps réel de l'objet satisfait la condition.
